(19) Europäisches Patentamt
European Patent Office
Office européen des brevets

(11) Publication number: 0 100 250 B1

(12) EUROPEAN PATENT SPECIFICATION

(45) Date of publication of patent specification: 04.03.87

(51) Int. Cl.⁴: C 07 D 311/30, A 61 K 31/35

(21) Application number: 83401211.4

(22) Date of filing: 13.06.83

# (54) Methylflavone derivatives.

(30) Priority: 23.06.82 JP 108928/82

(43) Date of publication of application:
08.02.84 Bulletin 84/06

(45) Publication of the grant of the patent:
04.03.87 Bulletin 87/10

(84) Designated Contracting States:
CH DE FR GB IT LI

(56) References cited:
EP-A-0 072 620
DE-A-2 051 269
DE-B-1 518 117
FR-M- 4 092
US-A-2 921 070

Chemical Abstracts col. 89, no. 13, 25 September 1978, Columbus, Ohio (US); G.DORIA et al.: "Antiallergic agents I. Substituted 4-oxo4H-1-benzopyran-6-carboxylic acids", p. 866, abstract 108943m

(73) Proprietor: NIPPON SHINYAKU COMPANY, LIMITED
14, Kisshoin Nishinosho Monguchicho
Minami-ku Kyoto-shi Kyoto (JP)

(72) Inventor: Enomoto, Hiroshi
26-3-7-707 Baba Miba Hashiri
Nogaokakyo-shi Kyoto-fu 617 (JP)
Inventor: Nomura, Tadatoshi
39-905 Okurayama Kobata
Uji-shi Kyoto-fu 611 (JP)
Inventor: Aoyagi, Yoshiaki
8-7-105 Uchidehama
Otsu-shi Shiga-ken 520 (JP)
Inventor: Chokai, Shoichi
1-57 Yamamoto Nakaaraita Sasa-cho
Kameoka-shi Kyoto-fu 621 (JP)
Inventor: Murase, Masao
439-11 Nomuracho
Kusatsu-shi Shiga-ken 525 (JP)
Inventor: Inoue, Kichiro
346-12 Ninomarucho Mukaijima
Fushimi-ku Kyoto 612 (JP)
Inventor: Shirahase, Ichiro
39 Oyake Sakanotsujicho
Yamashina-ku Kyoto 607 (JP)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

(74) Representative: **Hranitzky, Wilhelm Max et al**
**NOVAPAT - CABINET CHEREAU 5, Place du Molard**
**CH-1204 Genève (CH)**

**Description**

The present invention relates to novel 3-methyl-flavone derivatives represented by the following general formula (I) and salts thereof:

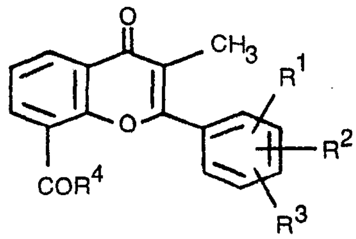

(I)

as well as manufacture methods therefor and pharmaceutical agents containing them as main ingredients(s).

In the formula, $R^1$, $R^2$ and $R^3$ are same or different and are hydrogen, halogen, alkyl with one to ten carbons, or alkoxy with one to four carbons; $R^4$ is hydroxyl, alkoxy with one to three carbons, hydroxyalkyloxy with one to three carbons or

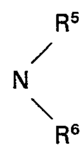

(in which $R^5$ is hydrogen or alkyl with one to seven carbons; $R^6$ is alkyl with one to seven carbons or cycloalkyl with five to seven members; the case where $R^5$ and $R^6$ are bonded together forming a five to seven membered ring is included).

The present invention compounds exhibit marked antiallergic, anti-inflammatory and expectorant action and are useful as remedies for asthma, allergic diseases, and inflammation.

The compounds of this invention exhibit marked characteristics such that they can be administered orally while conventional similar agents do not and further that their pharmaceutical actions are long acting.

For instance, sodium cromoglicate recently developed is reported by Cox et al to be effective for allergic asthma (cf. Advances in Drug Research, volume 5, page 115, 1970). It is supposed that this compound inhibits emission of chemical mediators from mast cells. Unfortunately this compound has a disadvantage that it does not show any pharmaceutical effect by oral administration and further that duration of its action is short.

Recently it has been known that SRS—A (slow reacting substance of anaphylaxia) which is one of chemical mediators plays a main role at the onset of asthma and, under such a situation, development of new and specific pharmaceuticals antiagonizing the action of SRS—A and exhibiting bio-synthesis inhibition action has been expected.

The present inventors during research and development of pharmaceuticals which satisfy the above objects, planned to develop pharamceuticals which are effective by oral administration, exhibit long acting action, and exhibit strong SRS—A antagonizing and synthesis inhibiting action, and, fortunately, have accomplished the present invention.

The present ivnention compounds are represented by the general chemical formula (I) as already given. Examples of alkyls applicable are methyl, ethyl, propyl, isopropyl, butyl, isobutyl, tertiary butyl, pentyl, hexyl, heptyl and octyl. Examples of halogen are fluorine, chlorine, bromine and iodine.

Examples of alkoxy are methoxy, ethoxy, propoxy, isopropoxy butoxy and tertiary butoxy. Examples of hydroxyalkyloxy are hydroxymethyloxy, betahydroxyethyloxy and gamma-hydroxypropyloxy.

Examples of

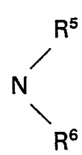

are methylamino, ethylamino, propylamino, isopropylamino, butylamino, tertiary butylamino, pentyl-amino, hexylamino, cyclopentylamino, cyclohexylamino, cycloheptylamino, dimethylamino, ethyl methylamino, diethylamino, methyl propylamino, ethyl propylamino, dipropylamino, butyl methylamino, butyl

ethylamino and dibutylamino. Examples where $R^5$ and $R^6$ are bonded to form a ring are pyrrolidino, piperidino and morpholino.

Representative examples of the present invention compounds are as follows:

Methyl 3 - methylflavone - 8 - carboxylate, 3 - methylflavone - 8 - carboxylic acid N,N - diethylamide, 2′ - methoxy - 3 - methylflavone - 8 - carboxylic acid, ethyl 2′ - methoxy - 3 - methylflavone - 8 - carboxylate, 2′-methoxy - 3 - methylflavone - 8 - carboxylic acid N,N - diethylamide, 2′ - methoxy - 3 - methylflavone - 8 - carboxylic acid N-ethylamide, 3′-methoxy - 3 - methylflavone - 8 - carboxylic acid, 4′ - methoxy - 3 - methylflavone - 8 - carboxylic acid, ethyl 4′ - methoxy - 3 - methylflavone - 8 - carboxylate, 4′ - methoxy - 3 - methylflavone - 8 - carboxylic acid N,N - diethylamide, 4′ - methoxy - 3 - methylflavone - 8 - carboxylic acid N - ethylamide, 2′ - ethoxy - 3 - methylflavone - 8 - carboxylic acid, ethyl 2′ - ethoxy - 3 - methylflavone - 8 - carboxylate, beta - hydroxyethyl 2′ - ethoxy - 3 - methylflavone - 8 - carboxylate, 2′ - ethoxy - 3 - methylflavone - 8 - carboxylic acid N,N - diethylamide, 2′ - ethoxy - 3 - methylflavone - 8 - carboxylic acid N - ethylamide, 2′ - isopropoxy - 3 - methylflavone - 8 - carboxylic acid, ethyl 2′ - isopropoxy - 3 - methylflavone - 8 - carboxylate, 2′ - isopropoxy - 3 - methylflavone - 8 - carboxylic acid N,N - diethylamide, 2′,3′ - dimethoxy - 3 - methylflavone - 8 - carboxylic acid, ethyl 2′,3′ - dimethoxy - 3 - methylflavone - 8 - carboxylate, 3′,4′ - dimethoxy - 3 - methylflavone - 8 - carboxylic acid, 2′,4′ - dimethoxy - 3 - methylflavone - 8 - carboxylic acid, ethyl 2′,4′ - dimethoxy - 3 - methylflavone - 8 - carboxylate, 2′,4′ - dimethoxy - 3 - methylflavone - 8 - carboxylic acid N,N - diethylamide, 2′,3′,4′ - trimethoxy - 3 - methylflavone - 8 - carboxylic acid, ethyl 2′,3′,4′ - trimethoxy - 3 - methylflavone - 8 - carboxylate, 2′,3′,4′ - trimethoxy - 3 - methylflavone - 8 - carboxylic acid N,N - diethylamide, 3′,4′,5′ - trimethoxy - 3 - methylflavone - 8 - carboxylic acid, 2′,3 - dimethylflavone - 8 - carboxylic acid, 2′,3 - dimethylflavone - 8 - carboxylic acid ethyl ester, 2′,3 - dimethylflavone - 8 - carboxylic acid N,N - diethylamide, 2′ - ethyl - 3 - methylflavone - 8 - carboxylic acid, ethyl 2′ - ethyl - 3 - methylflavone - 8 - carboxylate, 2′ - ethyl - 3 - methylflavone - 8 - carboxylic acid N,N - diethylamide, 2′,3,4′ - trimethylflavone - 8 - carboxylic acid, ethyl 2′,3,4′ - trimethylflavone - 8 - carboxylate, beta - hydroxyethyl 2′,3,4′-trimethylflavone - 8 - carboxylate, 2′,3,4′ - trimethylflavone - 8 - carboxylic acid N,N - diethylamide, 2′,3,4′ - trimethylflavone - 8 - carboxylic acid N - n - butyl - N - ethylamide, 4′ - ethyl - 3 - methylflavone - 8 - carboxylic acid, ethyl 4′ - ethyl - 3 - methylflavone - 8 - carboxylate, 4′ - ethyl - 3 - methylflavone - 8 - carboxylic acid N,N - diethylamide, 4′ - isopropyl - 3 - methylflavone - 8 - carboxylic acid, ethyl 4′ - isopropyl - 3 - methylflavone - 8 - carboxylate, 4′ - isopropyl - 3 - methylflavone - 8 - carboxylic acid N - ethylamide, 4′ - isopropyl - 3 - methylflavone - 8 - carboxylic acid N-n-hexylamide, 4′ - isopropyl - 3 - methylflavone - 8 - carboxylic acid N - cyclohexylamide, 4′ - isopropyl - 3 - methylflavone - 8 - carboxylic acid N,N - diethylamide, 4′ - isopropyl - 3 - methylflavone - 8 - carboxylic acid N - n - butyl - N - ethylamide, 4′ - isopropyl - 3 - methylflavone - 8 - carboxylic acid N,N - di - n - butylamide, 4′ - isopropyl - 3 - methylflavone - 8 - carboxylic acid N,N - pentamethylene amide, 4′ - tertiary butyl - 3 - methylflavone - 8 - carboxylic acid, ethyl 4′ - tertiary butyl - 3 - methylflavone - 8 - carboxylate, 4′ - tertiary butyl - 3 - methylflavone - 8 - carboxylic acid N,N - diethylamide, 3 - methyl - 4′ - n - pentylflavone - 8 - carboxylic acid, ethyl 3 - methyl - 4′ - n - pentylflavone - 8 - carboxylate, 3 - methyl - 4′ - n - pentylflavone - 8 - carboxylic acid N,N - diethylamide, 3 - methyl - 4′ - n - octylflavone - 8 - carboxylic acid, ethyl 3 - methyl - 4′ - n - octylflavone - 8 - carboxylate, 3 - methyl - 4′ - n - octylflavone - 8 - carboxylic acid N,N - diethylamide, 3 - methyl - 4′ - n - octylflavone - 8 - carboxylic acid N - ethylamide, 2′ - chloro - 3 - methylflavone - 8 - carboxylic acid, ethyl 2′ - chloro - 3 - methylflavone - 8 - carboxylate, beta - hydroxyethyl - 2′ - chloro - 3 - methylflavone - 8 - carboxylate, 4′ - chloro - 3 - methylflavone - 8 - carboxylic acid, ethyl 4′ - chloro - 3 - methylflavone - 8 - carboxylate, beta - hydroxyethyl 4′ - chloro - 3 - methylflavone - 8 - carboxylate, 4′ - chloro - 3 - methylflavone - 8 - carboxylic acid N,N - diethylamide, 4′ - chloro - 3 - methylflavone - 8 - carboxylic acid N-ethylamide, 2′,4′ - dichloro - 3 - methylflavone - 8 - carboxylic acid, ethyl 2′,4′ - dichloro - 3 - methylflavone - 8 - carboxylate, 2′,4′ - dichloro - 3 - methylflavone - 8 - carboxylic acid N,N - diethylamide, 2′,5′ - dichloro - 3 - methylflavone - 8 - carboxylic acid, ethyl 2′,5′ - dichloro - 3 - methylflavone - 8 - carboxylate, 2′,5′ - dichloro - 3 - methylflavone - 8 - carboxylic acid N,N - diethylamide.

3-Methylflavone derivatives according to the present invention can be manufactured by various routes as shown below.

Method 1

United States Patent No. 2921070, Japan Examined Patent Publication Sho-41-7953.

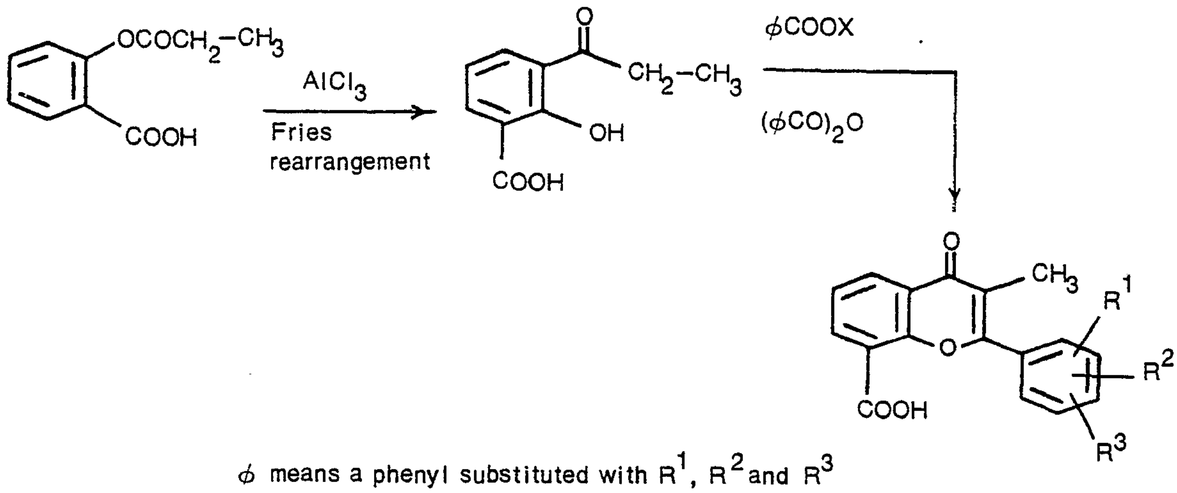


ϕ means a phenyl substituted with $R^1$, $R^2$ and $R^3$

X means alkali metal

Thus, 2-propionyloxybenzoic acid is heated with anhydrous aluminum chloride to subject to Fries rearrangement and the resulting 3-carboxy-2-hydroxypropiophenone is heated with benzoic anhydride and alkali benzoate at 180 to 190°C to afford 3-methylflavone-8-carboxylic acid derivatives.

Method 2

Japan Unexamined Patent Publication Sho-49-80035.

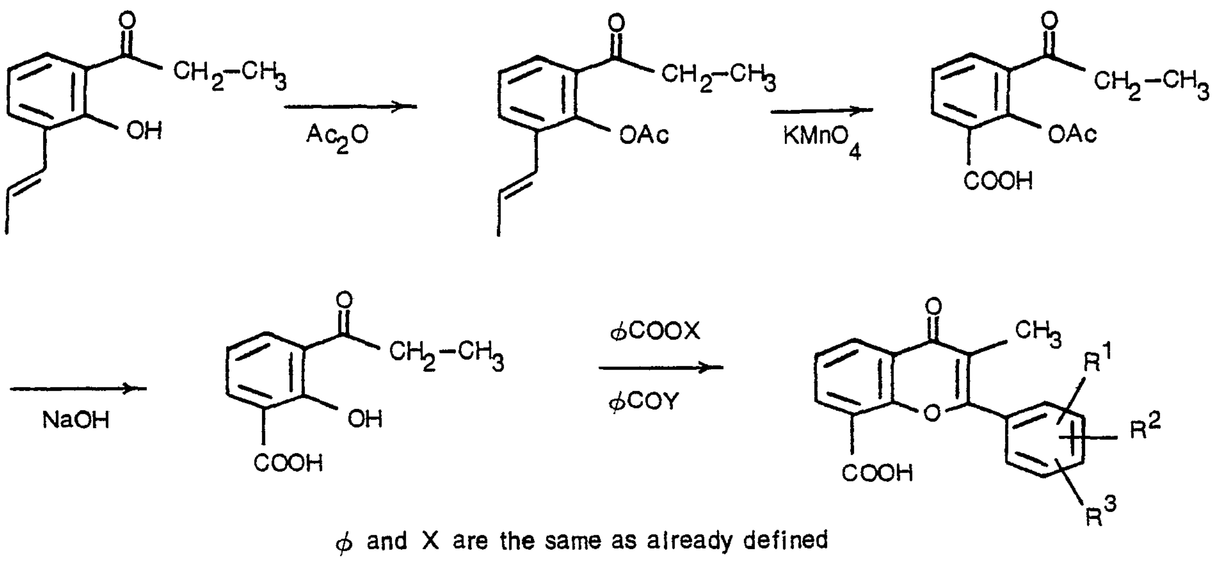


ϕ and X are the same as already defined

Y is halogen; Ac is acetyl

Thus 2-hydroxy-3-propenylpropiophenone is acetylated, oxidized with a suitable oxidizing agent such as potassium permanganate, the resulting carboxylic acid is deacetylated, and heated with benzoyl halide and alkali benzoate at 185 to 195°C to give 3-methylflavone-8-carboxylic acid derivatives.

Method 3

Japan Examined Patent Publication Sho-51-6150.

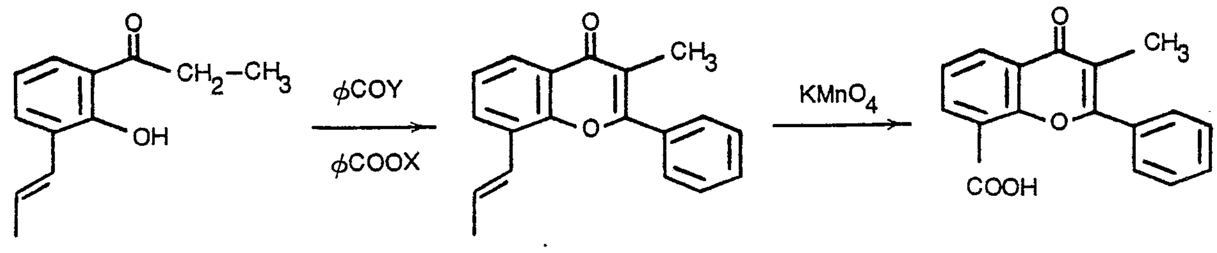


(Meanings of the symbols used are the same as already defined)

Thus, 2-hydroxy-3-propenylpropiophenone is heated with benzoyl halide and alkali benzoate at 180 to 190°C and the resulting 3-methyl-8-propenylflavone derivative is oxidized with an oxidizing agent such as potassium permanganate to afford 3-methylflavone-8-carboxylic acid derivatives.

In the methods 1 to 3, sodium benzoate is used as a base in the ring closure reaction to form methylflavone. When the above ring closure method is applied in synthesizing 3-methylflavone derivatives having various substituent(s) at 2-phenyl, some benzoic acid derivatives with substituent(s) are very expensive and the yield of the products may be very poor depending upon the substituent(s) applied. Further, the recovery rate of benzoic acid after the reaction is comparatively poor.

In order to improve such disadvantages, the present inventors have conducted various tests and succeeded in synthesizing various kinds of 3-methylflavone derivatives having substituted phenyl at 2-position by way of the following routes.

Method 4

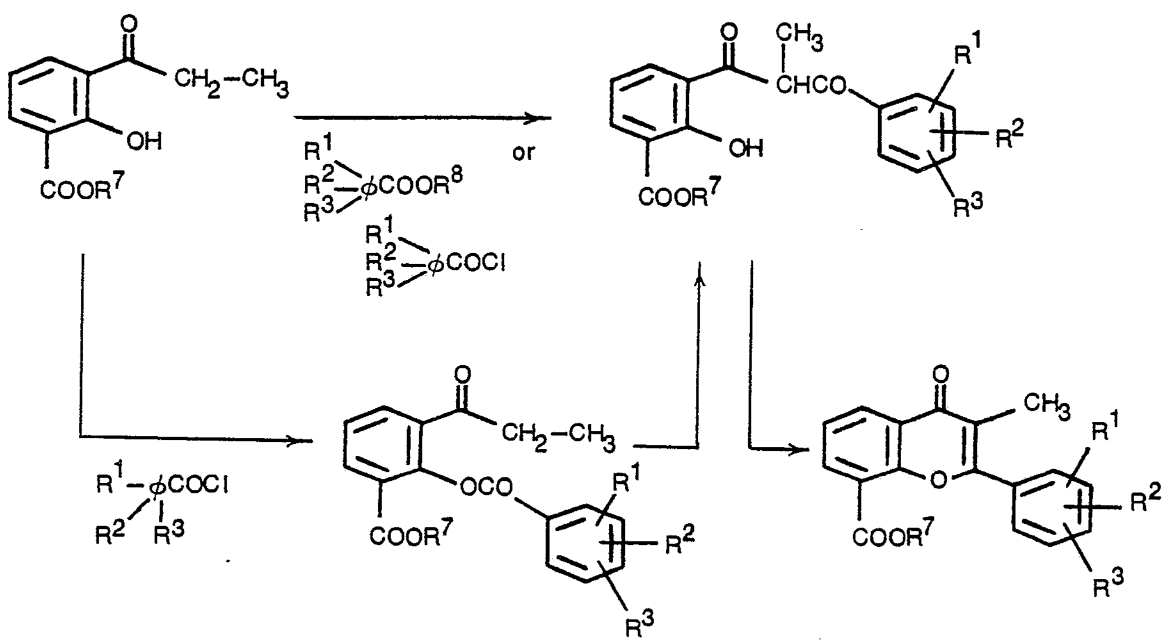


In the formulae, meanings of $R^1$, $R^2$ and $R^3$ are the same as those already defined and $R^7$ and $R^8$ stand for lower alkyl such as methyl, ethyl, propyl and butyl.

Method 5

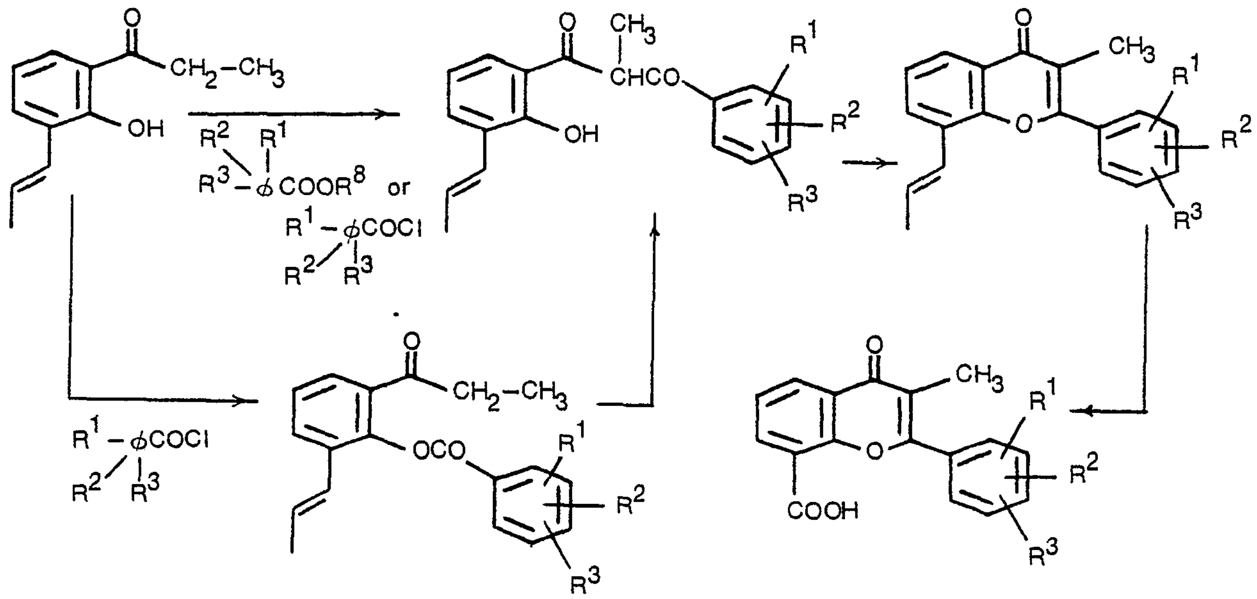

In the formulae, meanings of $R^1$, $R^2$, $R^3$ and $R^8$ are the same as those already defined.

With reference to the methods 4 and 5, detained illustrations are as follows.

Method 4

3-Propionylsalicylate is made to react with substituted benzyl chloride or with substituted benzoate at 50 to 100°C for two to eight hours in the presence of two or more equivalents (preferably, 2.1 to 6 equivalents) of base (such as, for example, sodium hydride or sodium alkoxides) in a suitable solvent (such as, for example, dimethylformamide, tetrahydrofuran, or dioxan) to afford 3-(alpha-substituted benzoyl-propionyl)-salicylate.

This compound may also be prepared by the following way. Thus, 3-propionylsalicylate is made to react with substituted benzoyl chloride at −10°C to room temperature for one to twenty-four hours in the presence of suitable amount of base (such as, for example, sodium hydride, sodium alkkoxides, potassium carbonate, pyridine or triethylamine) in a suitable solvent (such as, for example, acetone, acetonitrile or pyridine) to afford o-substituted benzoyl-3-propionylsalicylate. Depending upon the solvent used, this intermediate may be isolated therefrom but, in most cases, this is heated, without isolation therefrom, with suitable amount of base (such as, for example, sodium hydride, sodium alkoxide or potassium carbonate) at 50 to 130°C for one to eight hours.

Anyway, the resulting 3-(alpha-substituted benzoylpropionyl)-salicylate is treated in 1 to 40% ethanolic hydrochloric acid for one to three hours at room temperature or under heating to reflux to afford 3-methyl-flavone-8-carboxylate derivatives.

Method 5

2-Hydroxy-3-propenylpropiophenone is made to react with substituted benzoyl chloride or with substituted benzoate at 50 to 100°C for two to eight hours in the presence of two or more equivalents (preferably 2.1 to 6 equivalents) of base (such as, for example, sodium hydride or sodium alkoxide) in a suitable solvent (such as, for example, dimethyl formamide, dioxan or tetrahydrofuran) to afford 2-hydroxy-3-propenyl-alpha-substituted benzoyl propiophenone.

These intermediates may alternatively be prepared by the following way. Thus, 2-hydroxy-3-propenyl-propiophenone is made to react with substituted benzoyl chloride at −10°C to room temperature for one to twenty-four hours in the presence of suitable amount of base (such as, for example, sodium hydride, sodium alkoxide, potassium carbonate, pyridine or triethylamine) in a suitable solvent (such as, for example, acetone tetrahydrofuran dioxan, dimethyl formamide, acetonitrile or pyridine) to give 2-substituted benzoyloxy-3-propenylpropiophenone. This may be isolated therefrom depending upon the kind of the solvent used but, in most cases, it is treated in, without isolating therefrom, a suitable amount of base (such as, for example, sodium hydride, sodium alkoxide or potassium carbonate) at 50 to 130°C for one to eight hours.

Anyway, the resulting 2-hydroxy-3-propenyl-alpha-substituted benzoyl propiophenone is treated in 1 to 40% ethanolic hydrochloric acid at room temperature or under heating to reflux for one to three hours to afford 3-methyl-8-propenylflavone derivatives. This is oxidized, in a solvent mainly composed of acetic acid, with oxidizing agent such as potassium permanganate or potassium periodate to afford 3-methylflavone-8-carboxylic acid derivatives (II).

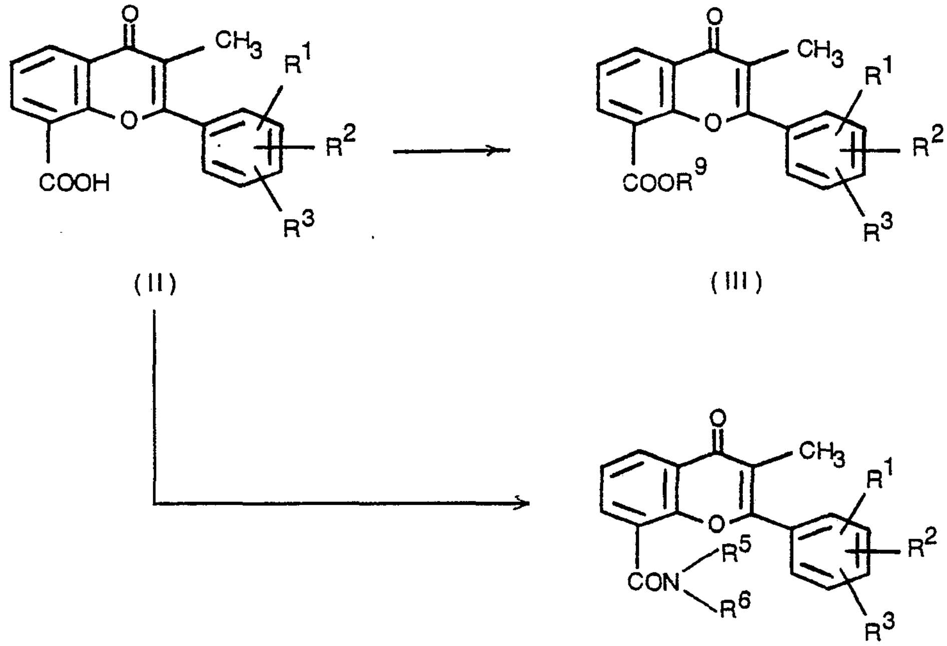

(II) (III) (IV)

In the formulae, meanings of $R^2$, $R^2$, $R^3$, $R^5$ and $R^6$ are the same as already defined and $R^9$ means lower alkyl (such as, for example, methyl, ethyl, propyl, isopropyl, butyl or tertiary butyl), hydroxyalkyl (such as, for example, hydroxymethyl, beta-hydroxyethyl or gamma-hydroxypropyl).

3-Methylflavone-8-carboxylic acid derivatives (II) as obtained above can be derived to 3-methylflavone-8-carboxylate derivatives (III) by various methods as shown below.

1. Acid halide method.

The carboxylic acid (II) which is a starting material or salt thereof is made to react with acid halogenating reagent (such as, for example, thionyl chloride, phosphorus trichloride, phosphorus pentachloride or phosphorus oxychloride) at 0 to 100°C for thirty minutes to ten hours in the presence or absence or organic solvent (such as, for example, methylene chloride, chloroform, benzene, toluene or xylene) to afford carboxylic acid halide. The resulting acid halide is made to react with alcohol (such as, for example, methanol, ethanol, propanol, isopropanol, butanol or tertiary butanol) at −10 to 100°C for one to ten hours in a suitable solvent (such as, for example, methylene chloride, chloroform, benzene, toluene, xylene, dioxan, acetone or dimethyl formamide) in the presence, if desired, of base (such as, for example, triethylamine, dimethylaniline, pyridine, sodium hydroxide, potassium hydroxide, potassium carbonate or sodium hydride) to give 3-methyl-flavone-8-carboxylic acid esters (III).

2. Direct esterification method.

The compound (II) or salt thereof is made to react with alcohol in the presence of an acid catalyst (such, as, for example, hydrochloric acid or sulfuric acid) to afford esters. Alternatively, the compound (II) and alcohol are subjected to dehydrative condensation in the presence of a condensation agent (such as, for example, dicyclohexyl carbodiimide, diethyl phosphoryl cyanide or diphenyl phosphoryl azide) to afford esters (III).

3. Acid anhydride method

The compound (II) is made to react with chlorocarbonates and the resulting mixed acid anhydride is made to react with alcohol to afford esters (III).

4. Actiated ester method

The compound (II) is made to react with 2,4-dinitrophenol, n-hydroxysuccinimide and the like and the resulting activated ester is made to react with alcohol to give esters (III).

5. Alkyl halide method

Metal salt of the compound (II) (such as sodium salt or potassium salt) or amine salt of the compound (II) (such as triethylamine salt and other amine salts) is made to react with alkyl halide to afford esters (III).

Incidentally, 3-methylflavone-8-carboxylic acid (II) can be derived to various 3-methylflavone-8-carboxylic acid amide derivatives (IV) by the following routes.

1. Acid halide method

The carboxylic acid (II) or salt thereof is made to react with acid halogenating agent (such as, for example, thionyl chloride, phosphorus trichloride, phosphorus pentachloride or phosphorus oxychloride) in the presence or absence of an organic solvent (such as, for example, methylene chloride, chloroform, benzene, toluene or xylene) at 0 to 100°C for thirty minutes to ten hours to afford carboxylic acid halide. The resulting acid halide is made to react with various amines (such as, for example, ammonia, methylamine, ethylamine, propylamine, isopropylamine, butylamine, tertiary butylamine, pentylamine, hexylamine, cyclopentylamine, cyclohexylamine, cycloheptylamine, dimethylamine, ethyl methylamine, diethylamine, methyl propylamine, ethyl propylamine, dipropylamine, butyl methylamine, butyl ethylamine, dibutylamine, pyrrolidine, piperidine, morpholine, thiomorpholine or piperazine) at −10 to 100°C for one to ten hours in a suitable solvent (such as, for example, methylene chloride, chloroform, benzene, toluene, xylene, dioxan, tetrahydrofuran, acetone, methyl ethyl ketone or dimethyl formamide) in the presence or absence of base (such as, for example, triethylamine, dimethylaniline, pyridine, sodium hydroxide, potassium hydroxide, potassium carbonate, sodium carbonate or sodium hydride) to afford 3-methylflavone-8-carboxylic acid amide derivatives (IV).

2. Dehydrative condensation method

The compound (II) or salt thereof is subjected to dehydrative condensation with an amine in the presence of a condensation agent (such as, for example, dicyclohexylcarbodiimide, diethylphosphoryl cyanide or diphenylphosphoryl azide) to synthesize carboxylic acid amide (IV).

3. Acid anhydride method

The compound (II) or salt thereof is made to react with chlorocarbonate and the like and the resulting mixed acid anhydride is made to react with amine to afford carboxylic acid amide (IV).

4. Activated ester method

The compound (II) is made to react with 2,4-dinitrophenol, N-hydroxysuccinimide and the like to afford activated ester and then made to react with amines to afford carboxylic acid amides (IV).

The 3-methylflavone derivatives as manufactured above can be easily isolated and purified by conventional methods such as, for example, recrystallization or chromatography.

Among the present invention compounds thus prepared, those in which $R^4$ is hydroxy can be made into salts with conventional pharmaceutically acceptable basic compounds. Examples of such basic compounds are sodium hydroxide, potassium hydroxide, aluminum hydroxide, potassium hydroxide, sodium carbonate, potassium carbonate, sodium bicarbonate, potassium bicarbonate and other inorganic basic compounds, morpholine, piperazine, pyrrolidine, thiomorpholine, methylamine, diethylamine, butyl ethylamine, triethylamine and other organic basic compounds.

Compounds according to the present invention exhibit antiallergic action and, accordingly, they are possibly administered to patients suffering from asthma, hay fever (nose allergy), hives, atopic dermatitis, and the like. Further, they exhibit antiinflammatory action and, consequently, they can be adminminstered to patients suffering from chronic rheumatoid arthritis, pain after operations, acute inflammation of upper respiratory, toothache, dismenorrhea, and the like.

Effects of the present invention compounds as remedies for asthma and allergic diseases were evaluated by a passive cutaneous anaphylaxis assay (PCA) in rats and by measuring anti-SRS-A action using ileus of guinea pigs.

Test Method No. 1 (PCA)

(i) Antiserum abundant in homocytotropic antibody is prepared by the same method as Tada and Okumura did (cf. Journal of Immunology, volume 106, page 1002, 1976). Thus, one mg (calculated as an amount of protein) of DNP-As (2,4-dinitrophenyl-coupled ascaris extract) prepared by methods of Strejan and Campbell (Journal of Immunology, vol. 98, page 893, 1967) and of Eisen (Journal of American Chemical Society, volume 75, page 4593, 1953) and $1 \times 10^{-10}$pertussis vaccine are administered to each paw of Wister strain rats (180 to 200 grams body weights) by dividing the dose by four. Five days later, 0.4 mg of DNP—As is administered into muscle of back. Eight days later from the initial immunization, blood is taken from descending aorta under anesthetizing with ether, the resulting serum is stored at −80°C and is melted before use.

(ii) Effect of tested compounds is investigated as follows:

Anti-serum obtained by the method (i) is diluted with physiological saline solution double by double successively and 0.05 ml of each diluted solution is administered into back of Wister strain rats (140 to 160 grams body weight) subcutaneously. After 72 hours, a solution of 2 mg (calculated as protein) of DNP—As and 2.5 mg of Evans Blue dissolved in 1 ml of physioligical saline solution is administered intravenously at a dose of 5 ml/kg. After thirty minutes from antigen solution administration, the animals are killed and diameters of blue spots appeared at the place where antiserum is administered are measured. The PCA test is conducted by the same method as already described using diluted solutions of antiserum which always show 10 mm or more of spot diameters and the effect of the test compounds is judged. Thus, antiserum diluted solutions are administered to two places in back. Test compounds are administered orally at the

dose of 10 mg/kg one hour before administration of antigen solution. From the skin of reacted parts of killed animals, leaked or emitted dyestuff is extracted and the amount of the dyestuff is measured. The inhibition ratio is calculated by the following expression:

$$\text{Inhibition Ratio} = (1 - \frac{A'}{A}) \times 100$$

in which R' is an amount of dyestuff in the group treated with the test compounds and A is that in the control group.

Anti-SRS—A action (An anti-action against slow reacting substance of anaphylaxis)

Hartley strain male guinea pigs (300 to 350 grams body weight) are killed and 1.0 to 1.5 cm of ileus is immediately excised from ileocecal parts and is suspended in 10 ml of Tyrode solution (95% $o_2$—5% $CO_2$ saturation) containing $10^{-7}$ g/ml of antopine and $10^{-6}$ g/ml of pyrilamine. SRA—A (20 units) (the amount of SRS—A showing the same shrinkage as 5 ng of histamine is defined as one unit) prepared by using sensitized guinea pig lung is given to cause shrinkage there. The antagonistic action of test compounds treated five minutes ago against the shrinkage is measured and recorded via isotonic transducer.

$$\text{Inhibition Ratio of Test Compound (\%)} = (1 - \frac{A'}{A}) \times 100$$

in which A' is a height of shrikage of SRS—A + test compound and A is that of SRS—A.

TABLE 1

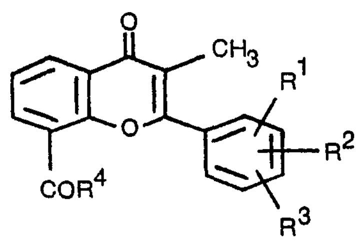

| EXAMPLE, No | $R^1$, $R^2$, $R^3$ | $R^4$ | PCA INHIBITION RATIO % | SRS—A $10^{-6}$M | INHIBITION RATIO % $10^{-7}$M |
|---|---|---|---|---|---|
| 8 | H | OMe | 17.4 | 64.9 | 45.0 |
| 9 | H | N $(Et)_2$ | 21.9 | 64.7 | 50.7 |
| 11 | 2'-OMe | OEt | 16.3 | 100 | 35.1 |
| 12 | 2'-OMe | N $(Et)_2$ | 19.2 | 52.4 | 8.3 |
| 13 | 2'-OMe | NHEt | 23.0 | 45.2 | 37.4 |
| 15 | 4'-OMe | NHEt | 19.0 | 41.9 | 30.3 |
| 16 | 2'-OEt | OH | 18.6 | 14.8 | 10.4 |
| 17 | 2'-OEt | OEt | 21.1 | 72.5 | 50.0 |
| 18 | 2'-OEt | N $(Et)_2$ | 27.4 | 57.1 | 32.1 |
| 4 | 2'-OEt | $OCH_2CH_2OH$ | 20.5 | 67.2 | 42.4 |
| 19 | 2'-OEt | NHEt | 16.0 | 50.0 | 30.0 |
| 20 | 2'-O-iso$C_3H_7$ | OH | 20.7 | 38.5 | 20.4 |
| 21 | 2'-O-iso$C_3H_7$ | OEt | 17.8 | 31.0 | 19.8 |

| EXAMPLE No | $R^1$, $R^2$, $R^3$ | $R^4$ | PCA INHIBITION RATIO % | SRS—A 10$^{-6}$M | INHIBITION RATIO % 10$^{-7}$M |
|---|---|---|---|---|---|
| 22 | 2'-O-iso$C_3H_7$ | N $(Et)_2$ | 16.1 | 66.7 | 40.0 |
| 23 | 2',3'-$(OMe)_2$ | OH | 19.5 | 24.0 | 6.2 |
| 24 | 2',3'-$(OMe)_2$ | OEt | 20.4 | 100 | 18.2 |
| 28 | 2',4'-$(OMe)_2$ | N $(Et)_2$ | 20.1 | 63.2 | 23.0 |
| 31 | 3',4',5'-$(OMe)_3$ | OEt | 20.0 | 63.0 | 20.4 |
| 30 | 3',4',5'-$(OMe)_3$ | N( N $(Et)_2$ | [illegible] | 31.0 | 18.0 |
| 34 | 2'-Me | OEt | 19.5 | 71.4 | 47.6 |
| 35 | 2'-Me | N $(Et)_2$ | [illegible] 3 | 48.8 | 18.0 |
| 37 | 2'-Et | OEt | 20.5 | 100 | 25.0 |
| 38 | 2'-Et | N $(Et)_2$ | 16.0 | 100 | 29.6 |
| 39 | 2',4'-$(Me)_2$ | OH | 37.0 | 5.3 | 3.2 |
| 40 | 2',4'-$(Me)_2$ | OEt | 17.3 | 73.9 | 8.1 |
| 41 | 2',4'-$(Me)_2$ | N $(Et)_2$ | 31.4 | 54.1 | 23.4 |
| 42 | 2',4'-$(Me)_2$ | $OCH_2CH_2OH$ | 19.8 | 27.6 | 10.3 |
| 43 | 2',4'-$(Me)_2$ | 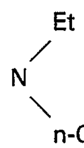 | 18.8 | 68.1 | 23.4 |
| 45 | 4'-Et | OEt | 19.0 | 52.2 | 16.0 |
| 7 | 4'-iso$C_3H_7$ | OH | 19.7 | 40.8 | 19.0 |
| 48 | 4'-iso$C_3H_7$ | N $(Et)_2$ | 27.2 | 100 | 23.3 |
| 49 | 4'-iso$C_3H_7$ | $N(n-C_4H_9)_2$ | 20.0 | 82.8 | 55.6 |
| 50 | 4'-iso$C_3H_7$ | 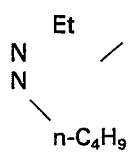 | 21.4 | 100 | 70.0 |
| 51 | 4'-iso$C_3H_7$ | NHEt | 19.0 | 75.0 | 17.5 |
| 52 | 4'-iso$C_3H_7$ | $NH(n-C_6H_{13})$ | 13.5 | 66.1 | 20.7 |
| 53 | 4'-iso$C_3H_7$ | NH(cyclo$C_6H_{11}$) | 10.3 | 51.9 | 40.0 |
| 54 | 4'-iso$C_3H_7$ | 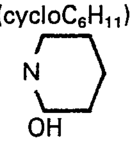 | 13.0 | 69.0 | 38.6 |
| 55 | 4'-tert$C_4H_9$ | OH | 20.3 | 100 | 21.1 |

| EXAMPLE No | $R^1$, $R^2$, $R^3$ | $R^4$ | PCA INHIBITION RATIO % | SRS—A $10^{-6}M$ | INHIBITION RATIO % $10^{-7}M$ |
|---|---|---|---|---|---|
| 56 | 4'-tert$C_4H_9$ | OEt | 20.2 | 44.9 | 19.0 |
| 57 | 4'-tert$C_4H_9$ | N $(Et)_2$ | 19.1 | 39.1 | 10.4 |
| 58 | 4'-n-$C_5H_{11}$ | OH | 28.8 | 40.0 | 10.3 |
| 59 | 4'-n-$C_5H_{11}$ | OEt | 19.4 | 38.1 | 9.8 |
| 60 | 4'-n-$C_5H_{11}$ | N $(Et)_2$ | [illegible].4 | 100 | 44.9 |
| 61 | 4'-n-$C_5H_{11}$ | ONa | 22.7 | 40.7 | 9.4 |
| 62 | 4'-n-$C_8H_{17}$ | OH | 16.3 | 83.3 | 31.0 |
| 67 | 2'-Cl | OEt | 19.8 | 100 | 42.9 |
| 68 | 2'-Cl | N $(Et)_2$ | 21.1 | 55.3 | 9.8 |
| 60 | 2'-Cl | $OCH_2CH_2OH$ | 14.3 | 55.3 | 9.8 |
| 71 | 4'-Cl | OEt | 36.9 | 10.3 | 6.0 |
| 72 | 4'-Cl | $N(Et)_2$ | 16.3 | 42.1 | 7.5 |
| 73 | 4'-Cl | NHEt | 19.4 | 29.4 | 6.2 |
| 74 | 4'-Cl | $OCH_2CH_2OH$ | 17.3 | 52.0 | 18.7 |
| 76 | 2',5'-$(Cl)_2$ | OH | 13.2 | 65.2 | 40.0 |
| 77 | 2',5'-$(Cl)_2$ | OEt | 16.3 | 67.4 | 6.9 |
| 78 | 2',5'-$(Cl)_2$ | N $(Et)_2$ | 26.3 | 79.3 | 5.6 |
| 80 | 2',4'-$(Cl)_2$ | OEt | 21.3 | 61.7 | 17.6 |
| 81 | 2',4'-$(Cl)_2$ | N $(Et)_2$ | 20.4 | 57.1 | 9.8 |

Antiinflammatory action was evaluated by measuring an inhibitory action of the compounds against carrageenin edema as illustrated below.

Carrageenin edema in hind foot of rat.

Experimental method:

SD strain rats of about 150 grams body weight were used; each group consisting of five rats. Thus, 0.1 ml of 0.5% carrgeenin solution in physiological saline water was hypodermically injected to right hind foot of rat. Test compound was given orally to rats one hour prior to the above carrageenin treatment. Then foot volumes before and three hours after the carrageenin treatment were measured and the difference was compared with that of the control group and used as a target of the pharmaceutical effect.

| Example Numbers | % Inhibition |
|---|---|
| 35 | 13.0 |
| 48 | 23.2 |
| 58 | 31.9 |
| 20 | 32.5 |
| 4 | 33.9 |
| 55 | 19.1 |
| Acetylsalicylic Acid (Control) | 31.6 |

Acute toxicity is observed for two weeks by oral administration of the test compounds to male mice. One group consists of four mice. Numbers of dead mice are shown in numerators.

| Example Number | Lethal Ratio |
|---|---|
| 4 | 0/4 |
| 11 | 0/4 |
| 13 | 1/4 |
| 18 | 0/4 |
| 48 | 0/4 |
| 58 | 0/4 |
| 62 | 0/4 |
| 74 | 0/4 |

Lethal ratios of all other compounds are 0/4 by oral administration of 3000 mg/kg.

The compounds of the present invention may be used for the treatment of allergic diseases, such as asthma, as follows: by oral administration, 1 to 1000 mg, one to three times daily; by rectal administration, 1 to 500 mg, one to three times daily: by inhalation, 0.1 to 100 mg, two to three times daily; by intraveneous administration, 0.1 to 50 mg, three to four times daily; by nasal administration, 0.1 to 100 mg, two to three times daily; by eye drops, 0.1 to 50 mg, three to four times daily; as an ointment, 1 to 100 mg, two to three times daily.

The present invention compounds can be applied to preparatory compositions by the following methods. Such preparatory compositions can be practically used using, if desired suitable pharmaceutical carrier or bulking agent, by conventional route. It is desired that those compositions are offered as a form adequate for being absorbed from stomach and intestinal canals. Still it is all right to administer them by non-oral route.

Examples of unit dose administration form are tablets, powders, fine particles, pills, granules and capsules. They may contain conventional vehicles or diluents such as binders (such as sirup, gum arabicum, gelatin, sorbitol, tragacanth, polyvinyl pyrrolidone, polyvinyl alcohol, hydroxypropyl methyl cellulose, hydroxypropyl cellulose, methyl cellulose or carboxymethyl cellulose sodium), diluents (such as lactose, sucrose, corn starch, calcium phosphate, sorbitol or crystalline cellulose), lubricants (such as magnesium stearate, talc, polyethyleneglycol or silica), disintegrating agents (such as potato starch, hydroxypropyl cellulose with lower degree of substitution, microcrystalline cellulose and the like), and acceptable wetting agents (such as sodium laurylsulfate).

Tablets may be subjected to coating by a known method.

Liquidal preparations may by oil suspensions, solutions, sirups, elixirs and the like and may be dried product which is redissolved in suitable vehicles such as water before use. Those liquid preparations may contain conventional additives. They are, for example, suspending agents such as methylcellulose, carboxymethylcellulose sodium, hydroxypropyl cellulose, hydroxypropyl methyl cellulose, polyvinyl pyrrolidone, polyvinyl alcohol, acacia, tragacanth, gelatin or sodium alginate; emulsifiers such as lecithin,

sorbitan, fatty acid esters, gum arabicum or tragacanth; wetting agents such as polyoxyethylene sorbitan fatty acid esters, polyoxyethylene fatty acid esters or polyoxyethylene derivatives of hydrogenated castor oil; nonaqueous vehicles such as sesame oil, soybean oil and other plant oils, propylene glycol, polyethylene glycol or ethyl alcohol; antiseptics such as methyl p-hydroxybenzoate, propyl p-hydroxybenzoate, or sorbic acid; sweetening agents such as simple sirup, sucrose, sorbitol, mannitol.

As to bases for rectal administration, fatty or oily bases such as cacao fat or triglyceride (Witepsol—Registered Trademark), or water-soluble bases such as polyethylene glycol can be used. The so-called rectal capsules in which the aqctive constituent is suspended in vegetable oil and made into gelatin capsules are also used.

Those preparations may be made into long acting preparations by known methods and forms or may be made into microcapsules.

It is desired that about 0.1 to 99% (or 0.5 to 90% in general) of one or more of the present invention effective compounds is contained in total compositions in the preparations.

Examples of manufacturing pharmaceutical preparations containing the present invention compounds as main constituents are given as hereunder.

Examples for pharmaceutical preparations.

(1) Capsules containing beta-hydroxyethyl 2′-ethoxy-3-methylflavone-8-carboxylate (compound of Example 4) as a main constituent.

The compound of Example 4 and diluents are uniformly mixed as per the following ratios and filled in hard gelatin capsules.

| | |
|---|---|
| Compound of Example 4 | 50 mg |
| Hydroxypropylcellulose of low degree of substitution | 20 mg |
| Lactose | 84 mg |
| Potato starch | 40 mg |
| Talc | 5 mg |
| Magnesium stearate | 1 mg |
| Mixed to make | 200 mg per capsule |

(2) Tablets mainly composed of 2′,3,4′-trimethylflavone-8-carboxylic acid N,N-diethylamide (the compound of Example 41).

A mixture of 100 mg of pulverized compound (of Example 41), 100 mg of lactose, 75 mg of crystalline cellulose and 40 mg of potato starch is mixed, kneaded after addition of binding agent solution prepared from 10 mg of polyvinyl alcohol, the mixture is passed through a sieve of 16 mesh to make granulated, dried, and passed through a sieve of 16 mesh once again to make the size of granules uniform. The granules are then mixed with 3 mg of magnesium stearate and 7 mg of talc and compressed into tablets. The resulting tablets may, if necessary, be coated with conventional coating base or with sucrose and the like.

The present invention is further illustrated by way of the following Reference Examples and Examples.

## Reference Example 1

A mixture of 225 grams of 2-hydroxypropiophenone, 247 grams of allyl bromide, 415 grams of potassium carbonate and 5 grams of potassium iodide is heated to reflux for twenth-four hours in 1 liter of acetone. After cooling, insoluble matters are removed by filtration, the filtrate is concentrated, the residue is dissolved in benzene, and the solution is washed with 10% sodium hydroxide solution. After washing with water, the solution is dried with anhydrous magnesium sulfate and benzene is evaporated therefrom. The resulting oil is distilled in vacuo to give 266 grams of 2-allyloxypropiophenone, colorless oil, b.p. 120 to 124°C.

2-Allyloxypropiophenone (266 grams) is heated at 230 to 240°C for six hours to conduct Claisen rearrangement. Then it is distilled in vacuo to give 233 grams of 3-allyl-2-hydroxypropiophenone, pale yellow oil, b.p. 112 to 115°C.

Then to 233 grams of 3-allyl-2-hydroxypropiophenone are added 212 grams of potassium hydroxide and 1100 ml of n-butanol and the mixture is heated to reflux for twenty-four hours. It is then poured into ice water, acidified with concentrated hydrochloric acid, crystals separated out therefrom are collected by filtration, washed with water and recrystallized from methanol to give 105 grams of 2-hydroxy-3-propenylpropiophenone, yellow needles, melting point 83 to 85°C.

Example 1

4'-Methoxy-3-methylflavone-8-carboxylic acid

To 2.47 grams of 2-hydroxy-3-propenylpripiophenone are added 6.65 grams of p-methoxybenzoyl chloride and one half of 6.88 grams of anhydrous sodium p-methoxybenzoate, the mixture is heated in a nitrogen stream and, when 2-hydroxy-3-propenylpropiophenone is dissolved, the residue of anhydrous sodium p-methoxybenzoate is added thereto, and the mixture is stirred at 180 to 190°C for five hours. After cooling, the mixture is dissolved in ethyl acetate, the soluiton is washed with 10% sodium hydroxide solution and with water, and dried with anhydrous magnesium sulfate and finally the solvent is evaporated therefrom to give 3.0 grams of 4'-methoxy-3-methyl-8-propenylflavone, colorless needles, melting point 169 to 171°C.

4'-Methoxy-3-methyl-8-propenylflavone (3 grams) is dissolved in 35 ml of glacial acetic acid, small amount of ice is added thereto, 6.8 grams of potassium permanganate is added thereto during eight hours keeping the solution at 20 to 30°C and stirring throughout, then aqueous solution of sodium bisulfite is added thereto keeping the solution at 30 to 40°C, and the mixture is stirred for one hour. Crystals separated out therefrom are collected by filtration, washed with water, then dissolved in 10% sodium hydroxide solution, the solution is treated with an activated charcoal, filtered, the filtrate is adjusted to pH 3 with concentrated hydrochloric acid, crystals separated out are collected by filtration, and washed with water to give 1.2 grams of 4'-methoxy-3-methylflavone-8-carboxylic acid, colorless needles, melting point 266 to 267°C.

Elementary analysis calculated for $C_{18}H_{14}O_5$: C 69.67, H 4.54; Found: C 69.74, H, 4.35.

Example 2

Ethyl 4'-methoxy-3-methylflavone-8-carboxylate

To 3 grams of 4'-methoxy-3-methylflavone-8-carboxylic acid is added 100 ml of 35% ethanolic hydrochloric acid, the mixture is heated to reflux for four hours, the solvent is evaporated therefrom, the residue is dissolved in ethyl acetate, the solution is washed with 10% sodium hydroxide solution, then with water, and dried with anhydrous magnesium sulfate. The solvent is evaporated therefrom and the residue is recrystallized from a mixture of benzene and n-hexane to give 2.7 grams of ethyl 4'-methoxy-3-methylflavone-8-carboxylate, colorless needles, melting point 137 to 139°C.

Elementary analysis calculated for $C_{20}H_{18}O_5$: C 70.99, H 5.36; Found: C 71.11, H 5.44.

Example 3

4'-Methoxy-3-methylflavone-8-carboxylic acid N,N-diethylamide

To 1.8 grams of 4'-methoxy-3-methylflavone-8-carboxylic acid is added 20 ml of thionyl chloride and the mixture is stirred at room temperature for seven hours. Thionyl chloride is evaporated therefrom, the residue is dissolved in 50 ml of chloroform, and 10 ml of diethylamine is dropped thereinto with ice cooling. The mixture is stirred at room temperature for two hours, washed with 10% hydrochloric acid, then with 10% sodium hydroxide solution, finally washed with water, and dried with anhydrous magnesium sulfate. The solvent is evaporated therefrom and the residue is recrystallized from a mixture of benzene and n-hexane to give 2.0 grams of 4'-methoxy-3-methylflavone-8-carboxylic acid N,N-diethylamide, colorless needles, melting point 143 to 147°C.

Elementary analysis calculated for $C_{22}H_{23}NO_4$: C 72.30, H 6.34; Found: C72.48, H 6.18.

Example 4

Beta-Hydroxyethyl 2'-ethoxy-3-methylflavone-8-carboxylate

One gram of 2'-ethoxy-3-methylflavone-8-carboxylic acid prepared by the same way as Example 1 is dissolved in 30 ml of dimethyl formamide, 0.5 gram of anhydrous potassium carbonate is added thereto, 2 grams of ethylene bromohydrin is dropped thereinto with stirring, and the mixture is stirred at 50°C for four hours. Then it is poured into ice water, the mixture is neutralized with 10% hydrochloric acid, extracted with ethyl acetate, the extract is washed with water, dried with anhydrous magnesium sulfate, the solvent is evaporated therefrom, and the residue is recrystallized from a mixture of benzene and n-hexane to give 0.8 gram of beta-hydroxyethyl 2'-ethoxy-3-methylflavone-8-carboxylate, colorless needles, melting point 143 to 144.5°C.

Elementary analysis calculated for $C_{21}H_{20}O_6$: C 68.46, H 5.47; Found: C 68.62, H 5.42.

Example 5

Sodium 2',3,4'-trimethylflavone-8-carboxylate

One gram of 2',3,4'-trimethylflavone-8-carboxylic acid prepared by the same way as Example 1 is dissolved in methanol, equimolar 2N sodium hydroxide solution is added thereto, methanol is evaporated therefrom, the residue is dissolved in small amount of water, the solution is lyophilized, and 1.0 gram of sodium 2',3,4'-trimethylflavone-8-carboxylate, colorless powder, melting point 218 to 220°C, is obtained.

Elementary analysis calculated for $C_{19}H_{15}O_4Na$: C 69.08, H 4.57; Found: C 68.87, H 4.64.

Example 6

N-n-Butyl-N-ethylamine salt of 4'-chloro-3-methylflavone-8-carboxylic acid

Two grams of 4'-chloro-3-methylflavone-8-carboxylic acid prepared by the same way as Example 1 is dissolved in 100 ml of chloroform. Butyl ethylamine (0.65 gram) is added thereto, then chloroform is evaporated therefrom and the residue is recrystallized from ethanol to give 2.2 grams of N-n-butyl-N-ethylamine salt of 4'-chloro-3-methylflavone-8-carboxylic acid, colorless flakes, melting point 184 to 189°C.

Elementary analysis calculated for $C_{23}H_{26}ClNO_4$: C 66.41, H 6.30, N 3.36; Found: C 66.36, H 6.38, N 3.26.

Example 7

4'-Isopropyl-3-methylflavone-8-carboxylic acid

2-Hydroxy-3-propyenylpropiophenone (2.4 grams) is dissolved in 30 ml of dimethylformamide and 0.6 gram of 50% sodium hydride is added thereto little by little at room temperature with stirring. The mixture is stirred for one hour more at the same temperature and 2.4 grams of p-isopropylbenzoyl chloride is dropped thereinto with ice cooling. The mixture is stirred at room temperature for one hour, 0.6 gram of 50% sodium hydride is added thereto, the mixture is heated at 60 to 70°C for one hour, cooled, and poured into ice water. This is extracted with ethyl acetate, the extract is washed with water, dried with anhydrous magnesium sulfate, the solvent is evaporated therefrom, and the resulting crystals are washed with n-hexane to give 3.6 grams of 2-hydroxy-3-propenyl-alpha-p-isopropylbenzoylpropiophenoner, pale yellow powder, melting point 108 to 110°C.

To 3.6 grams of the resulting 2-hydroxy-3-propenyl-alpha-p-isopropylbenzoylpropiophenone is added 10 ml of 40% ethanolic hydrochloric acid and the mixture is allowed to stand at room temperature for thirty minutes. This is poured into ice water, the mixture is neutralized with aqueous solution of sodium bicarbonate, extracted with ethyl acetate, the extract is washed with water, dried with anhydrous magnesium sulfate, the solvent is evaporated therefrom, the residue is washed with methanol and recrystallized from methanol to give 3.4 grams of 4'-isopropyl-3-methyl-8-propenylflavone, colorless prisms, melting point 105 to 107°C.

4'-Isopropyl-3-methyl-8-propenylflavone (3.4 grams) is dissolved in 40 ml of glacial acetic acid, small amount of water is added thereto, 6.6 grams of potassium permanganate is added thereto during eight hours with stirring keeping the inner temperature at 20 to 30°C, then aqueous solution of sodium bisulfite is added thereto keeping the inner temperature at 30 to 40°C, and the mixture is stirred for one hour. Crystals separated out therefrom are collected by filtration, washed with water, dissolved in 10% sodium hydroxide solution, the solution is treated with activated charcoal, filtered, and the filtrate is adjusted to pH 3 with concentrated hydrochloric acid, crystals separated out are collected by filtration, washed with water and recrystallized from methanol to give 1.9 grams of 4'-isopropyl-3-methylflavone-8-carboxylic acid, colorless prisms, melting point 216 to 220°C.

Elementary analysis calculated for $C_{20}H_{18}O_4$: C 74.51, H 5.62; Found: C 74.44, H 5.63.

Compounds of Examples 8 to 81 are synthesized by the same way as described for the compounds of Examples 1 to 7. They are given in the following tables.

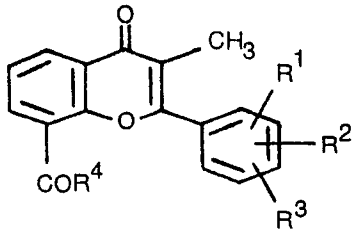

| Example No. | $R^1$, $R^2$, $R^3$ | $R^4$ | Appearance | M.p. (°C) | Expt./ Formula | Elementary Analysis (%) Upper column: Calcd. Lower column: Found |
|---|---|---|---|---|---|---|
| 8 | H | OMe | Colorless needles | 173~176 | $C_{18}H_{14}O_4$ | C 73.45 H 4.79<br>C 73.54 H 4.53 |
| 9 | H | N $(Et)_2$ | Colorless crystals | 164~169 | $C_{21}H_{21}NO_3$ | C 75.20 H 6.31 N 4.17<br>C 75.41 H 6.34 N 4.10 |
| 10 | 2′-OMe | OH | Colorless needles | 233~240 | $C_{18}H_{14}O_5$ | C 69.67 H 4.54<br>C 69.67 H 4.45 |
| 11 | 2′-OMe | OEt | Colorless needles | 91~93 | $C_{20}H_{18}O_5$ | C 70.99 H 5.36<br>C 71.16 H 5.10 |
| 12 | 2′-OMe | N $(Et)_2$ | Colorless crystals | 169~174 | $C_{22}H_{23}NO_4$ | C 72.30 H 6.34 N 3.83<br>C 72.09 H 5.29 N 3.78 |
| 13 | 2′-OMe | NHEt | Colorless needles | 178~179 | $C_{20}H_{19}NO_4$ | C 71.20 H 5.67 N 4.15<br>C 71.05 H 5.67 N 4.10 |
| 14 | 3′OMe | OH | Colorless needles | 228~229 | $C_{18}H_{14}O_5$ | C 69.67 H 4.54<br>C 69.57 H 4.41 |
| 15 | 4′-OMe | NHEt | Colorless needles | 217~221 | $C_{20}H_{19}NO_4$ | C 72.20 H 5.67 N 4.15<br>C 71.30 H 5.70 N 4.03 |
| 16 | 2′-OEt | OH | Colorless crystals | 213~215 | $C_{19}H_{16}O_5$ | C 70.36 H 4.97<br>C 70.34 H 5.00 |
| 17 | 2′-OEt | OEt | Colorless crystals | 118~120 | $C_{21}H_{20}O_5$ | C 71.57 H 5.72<br>C 71.82 H 5.64 |
| 18 | 2′-OEt | N $(Et)_2$ | Colorless crystals | 110~113 | $C_{23}H_{25}NO_4$ | C 72.80 H 6.64 N 3.69<br>C 72.83 H 6.67 N 3.66 |
| 19 | 2′-OEt | NHEt | Colorless needles | 154~157 | $C_{21}H_{21}NO_4$ | C 71.77 H 6.02 N 3.93<br>C 71.93 H 6.19 N 3.85 |
| 20 | 2′-O-iso$C_3H_7$ | OH | Colorless crystals | 222~225 | $C_{20}H_{18}O_5$ | c C 70.99 H 5.36<br>C 71.09 H 5.43 |
| 21 | 2′-O-iso$C_3H_7$ | OEt | Colorless crystals | 103~104 | $C_{22}H_{22}O_5$ | C 72.11 H 6.05<br>C 72.20 H 6.08 |
| 22 | 2′-O-iso$C_3H_7$ | N $(Et)_2$ | Colorless crystals | 129~133 | $C_{24}H_{27}NO_4$ | C 73.25 H 6.91 N 3.55<br>C 73.11 H 6.86 N 3.46 |
| 23 | 2′,3′-$(OMe)_2$ | OH | Colorless crystals | 255~260 | $C_{19}H_{16}O_6$ | C 67.05 H 4.73<br>C 67.07 H 4.63 |
| 24 | 2′,3′-$(OMe)_2$ | OEt | Pale yellow needles | 109~113.5 | $C_{21}H_{20}O_6$ | C 68.46 H 5.47<br>C 68.72 H 5.20 |

| Example No. | $R^1$, $R^2$, $R^3$ | $R^4$ | Appearance | M.p. (°C) | Expt./ Formula | Elementary Analysis (%) Upper column: Calcd. Lower column: Found |
|---|---|---|---|---|---|---|
| 25 | 3′,4′-(OMe)$_2$ | OH | Pale yellow needles | 273~274 | $C_{19}H_{16}O_6$ | C 67.05 H 4.73<br>C 67.31 H 4.58 |
| 26 | 2′,4′-(OMe)$_2$ | OH | Colorless crystals | 213~216 | $C_{19}H_{16}O_6$ | C 67.05 H 4.73<br>C 67.13 H 4.66 |
| 27 | 2′,4′-(OMe)$_2$ | OEt | Colorless needles | 145~149.5 | $C_{21}H_{20}O_6$ | C 68.46 H 5.47<br>C 68.69 H 5.51 |
| 28 | 2′,4′-(OMe)$_2$ | N (Et)$_2$ | Colorless crystals | 194~198 | $C_{23}H_{25}NO_5$ | C 69.85 H 6.37 N 3.54<br>C 69.99 H 6.36 N 3.37 |
| 29 | 2′,3′,4′-(OMe)$_3$ | OH | Colorless needles | 215~218 | $C_{20}H_{28}O_7$ | C 64.86 H 4.90<br>C 64.98 H 4.86 |
| 30 | 2′,3′,4′-(OMe)$_3$ | N (Et)$_2$ | Colorless needles | 154~157 | $C_{24}H_{27}NO_6$ | C 67.75 H 6.40 N 3.29<br>C 67.53 H 6.49 N 3.18 |
| 31 | 2′,3′,4′-(OMe)$_3$ | OEt | Colorless crystals | 141~143 | $C_{22}H_{22}O_7$ | C 66.32 H 5.57<br>C 66.34 H 5.56 |
| 32 | 3′,4′,5′-(OMe)$_3$ | OH | Colorless needles | 263~265 | $C_{20}H_{18}O_7$ | C 64.86 H 4.90<br>C 64.88 H 4.94 |
| 33 | 2′-Me | OH | Colorless crystals | 256.5~258 | $C_{18}H_{14}O_4$ | C 73.45 H 4.79<br>C 73.58 H 4.59 |
| 34 | 2′-Me | OEt | Colorless needles | 89~101 | $C_{20}H_{18}O_4$ | C 74.51 H 5.62<br>C 74.68 H 5.52 |
| 35 | 2′-Me | N (Et)$_2$ | Pale yellow needles | 134~136 | $C_{22}H_{23}NO_3$ | C 75.62 H 6.63 N 4.00<br>C 75.71 H 6.54 N 3.95 |
| 36 | 2′-Et | OH | Colorless crystals | 203~207 | $C_{19}H_{16}O_4$ | C 74.01 H 5.23<br>C 74.18 H 5.21 |
| 37 | 2′-Et | OEt | Colorless oil | — | $C_{21}H_{20}O_4$ | C 74.98 H 5.99<br>C 74.89 H 5.99 |
| 38 | 2′-Et | N (Et)$_2$ | Pale brown crystals | 117~120 | $C_{23}H_{25}NO_3$ | C 76.00 H 6.93 N 3.85<br>C 76.25 H 6.96 N 3.85 |
| 39 | 2′,4′-OMe)$_2$ | OH | Colorless crystals | 217~219 | $C_{19}H_{16}O_4$ | C 74.01 H 5.23<br>C 73.92 H 5.19 |
| 40 | 2′,4′-OMe)$_2$ | OEt | Colorless crystals | 90~92 | $C_{21}H_{20}O_4$ | C 74.98 H 5.99<br>C 75.16 H 5.88 |
| 41 | 2′,4′-(Me)$_2$ | N (Et)$_2$ | Colorless crystals | 108~111 | $C_{23}H_{25}NO_3$ | C 76.00 H 6.93 N 3.85<br>C 76.25 H 6.65 N 3.98 |
| 42 | 2′,4′-(Me)$_2$ | $OCH_2CH_2OH$ | Colorless crystals | 113~115 | $C_{21}H_{20}O_5$ | C 71.57 H 5.72<br>C 71.57 H 5.64 |
| 43 | 2′,4′-(Me)$_2$ | 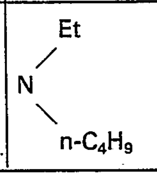 | Pale yellow crystals | 110~112 | $C_{25}H_{29}NO_3$ | C 76.69 H 7.46 N 3.57<br>C 76.44 H 7.65 N 3.62 |

| Example No. | $R^1$, $R^2$, $R^3$ | $R^4$ | Appearance | M.p. (°C) | Expt./ Formula | Elementary Analysis (%) Upper column: Calcd. Lower column: Found |
|---|---|---|---|---|---|---|
| 44 | 4'-Et | OH | Yellow crystals | 228~229.5 | $C_{19}H_{16}O_4$ | C 74.01 H 5.23<br>C 74.14 H 5.24 |
| 45 | 4'-Et | OEt | Yellow needles | 75~76.5 | $C_{21}H_{20}O_4$ | C 74.98 H 5.99<br>C 75.06 H 6.07 |
| 46 | 4'-Et | N.(Et)$_2$ | Colorless crystals | 145~147 | $C_{23}H_{25}NO_3$ | C 76.00 H 6.93 N 3.85<br>C 75.86 H 6.96 N 3.79 |
| 47 | 4'-iso$C_3H_7$ | OEt | Colorless needles | 57~59 | $C_{22}H_{24}O_4$ | C 75.40 H 6.32<br>C 75.48 H 6.38 |
| 48 | 4'-iso$C_3H_7$ | N (Et)$_2$ | Pale brown crystals | 136~139 | $C_{29}H_{27}NO_3$ | C 76.36 H 7.20 N 3.71<br>C 76.42 H 7.31 N 3.56 |
| 49 | 4'-iso$C_3H_7$ | N(n-$C_4H_9$)$_2$ | Colorless crystals | 104~106 | $C_{28}H_{35}NO_3$ | C 77.56 H 8.13 N 3.23<br>C 77.50 H 8.09 N 3.39 |
| 50 | 4'-iso$C_3H_7$ | 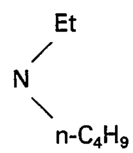 | Pale brown crystals | 113~115.5 | $C_{26}H_{31}NO_3$ | C 77.00 H 7.70 N 3.45<br>C 77.17 H 7.49 N 3.50 |
| 51 | 4'-iso-$C_3H_7$ | NHEt | Colorless crystals | 162~165 | $C_{22}H_{23}NO_3$ | C 75.62 H 6.63 N 4.00<br>C 75.44 H 6.62 N 3.96 |
| 52 | 4'-iso-$C_3H_7$ | NH(n-$C_5H_{13}$) | Colorless crystals | 146~148 | $C_{26}H_{13}NO_3$ | C 77.00 H 7.70 N 3.45<br>C 77.06 H 7.85 N 3.65 |
| 53 | 4'-iso-$C_3H_7$ | 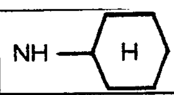 | Colorless crystals | 195~196.5 | $C_{26}H_{29}NO_3$ | C 77.38 H 7.24 N 3.47<br>C 77.54 H 7.34 N 3.54 |
| 54 | 4'-iso-$C_3H_7$ | 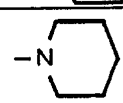 | Pale brown crystals | 162~165 | $C_{25}H_{27}NO_3$ | C 77.09 H 6.98 N 3.59<br>C 77.27 H 6.99 N 3.75 |
| 55 | 4'-tert-$C_4H_9$ | OH | Yellow crystals | 221~224 | $C_{21}H_{20}O_4$ | C 74.98 H 5.99<br>C 75.05 H 6.04 |
| 56 | 4'-tert-$C_4H_9$ | OEt | Colorless crystals | 97~99 | $C_{23}H_{24}O_4$ | C 75.80 H 6.63<br>C 75.85 H 6.73 |
| 57 | 4'-tert-$C_4H_9$ | N (Et)$_2$ | Colorless crystals | 145~148 | $C_{25}H_{29}NO_3$ | C 76.69 H 7.46 N 3.57<br>C 76.78 H 7.59 N 3.67 |
| 58 | 4'-n-$C_5H_{11}$ | OH | Colorless crystals | 202~206 | $C_{22}H_{22}O_4$ | C 75.40 H 6.32<br>C 75.35 H 6.14 |
| 59 | 4'-n-$C_5H_{11}$ | OEt | Colorless needles | 41~42 | $C_{24}H_{26}O_4$ | C 76.16 H 6.92<br>C 76.07 H 6.76 |
| 60 | 4'-n-$C_5H_{11}$ | N (Et)$_2$ | Colorless crystals | 102~103 | $C_{26}H_{31}NO_3$ | C 77.00 H 7.70 N 3.45<br>C 76.79 H 7.68 N 3.47 |
| 61 | 4'-n-$C_5H_{11}$ | ONa | White powder | 278~286 | $C_{22}H_{31}O_4Na$ | C 70.95 H 5.68<br>C 70.69 H 5.76 |

| Example No. | $R^1$, $R^2$, $R^3$ | $R^4$ | Appearance | M.p. (°C) | Expt./ Formula | Elementary Analysis (%) Upper column: Calcd. Lower column: Found |
|---|---|---|---|---|---|---|
| 62 | 4′-n-$C_5H_{11}$ | OH | Pale yellow crystals | 182~184 | $C_{25}H_{28}O_4$ | C 76.50 H 7.19<br>C 76.51 H 7.11 |
| 63 | 4′-n-$C_8H_{17}$ | OEt | Colorless oil | — | $C_{27}H_{32}O_4$ | C 77.11 H 7.66<br>C 77.10 H 7.78 |
| 64 | 4′-n-$C_8H_{17}$ | N $(Et)_2$ | Colorless oil | — | $C_{29}H_{37}NO_3$ | C 77.81 H 8.33 N 3.12<br>C 77.68 H 8.43 N 3.11 |
| 65 | 4′-n-$C_8H_{17}$ | NHEt | Colorless crystals | 128~129.5 | $C_{27}H_{33}NO_3$ | C 77.29 H 7.92 N 3.33<br>C 77.14 H 7.94 N 3.46 |
| 66 | 2′-Cl | OH | Colorless crystals | 281~284 | $C_{17}H_{11}ClO_4$ | C 64.87 H 3.52<br>C 64.92 H 3.32 |
| 67 | 2′-Cl | OEt | Colorless crystals | 80.5~82.5 | $C_{19}H_{15}ClO_4$ | C 66.57 H 4.41<br>C 86.77 H 4.22 |
| 68 | 2′-Cl | N $(Et)_2$ | Colorless crystals | 165~168 | $C_{21}H_{20}ClNO_3$ | C 68.19 H 5.45 N 3.78<br>C 68.32 H 5.51 N 3.66 |
| 69 | 2′-Cl | $OCH_2CH_2OH$ | Colorless crystals | 97.5~99.5 | $C_{19}H_{15}ClO_5$ | C 63.60 H 4.21<br>C 63.47 H 4.38 |
| 70 | 4′-Cl | OH | Colorless crystals | 259~260.5 | $C_{17}H_{11}ClO_4$ | C 64.87 H 3.52<br>C 65.01 H 3.44 |
| 71 | 4′-Cl | OEt | Pale yellow crystals | 136~139 | $C_{19}H_{15}ClO_7$ | C 66.57 H 4.41<br>C 66.86 H 4.13 |
| 72 | 4′-Cl | N $(Et)_2$ | Colorless crystals | 162~164.5 | $C_{21}H_{20}ClNO_3$ | C 68.19 H 5.45 N 3.78<br>C 68.28 H 5.36 N 3.67 |
| 73 | 4′-Cl | NHEt | Colorless needles | 249~251 | $C_{19}H_{16}ClNO_3$ | C 66.76 H 4.71 N 4.09<br>C 67.76 H 4.74 N 3.95 |
| 74 | 4′-Cl | $OCH_2CH_2OH$ | Colorless crystals | 184~187 | $C_{19}H_{15}ClO_5$ | C 63.60 H 4.21<br>C 63.78 H 4.11 |
| 75 | 4′-Cl | $OH{\cdot}Et_3N$ | Colorless prisms | 141~145 | $C_{23}H_{26}ClNO_4$ | C 66.41 H 6.30 N 3.36<br>C 66.53 H 6.33 N 3.48 |
| 76 | 2′,5′-$(Cl)_2$ | Oh | Colorless crystals | 300~??? | $C_{17}H_{10}Cl_2O_4$ | C 58.47 H 2.88<br>C 58.48 H 2.73 |
| 77 | 2′,5′-$(Cl)_2$ | OEt | Colorless crystals | 85~88.5 | $C_{19}H_{14}Cl_1O_4$ | C 60.49 H 3.74<br>C 60.74 H 3.58 |
| 78 | 2′,5′-$(Cl)_2$ | N $(Et)_2$ | Pale brown crystals | 185~187 | $C_{21}H_{19}Cl_2NO_3$ | C 62.38 H 4.73 N 3.46<br>C 62.62 H 4.67 N 3.30 |
| 79 | 2′,4′-$(Cl)_2$ | OH | Colorless crystals | 253.5~255 | $C_{17}H_{10}Cl_2O_4$ | C 58.47 H 2.88<br>C 58.66 H 2.70 |

| Example No. | $R^1$, $R^2$, $R^3$ | $R^4$ | Appearance | M.p. (°C) | Expt./ Formula | Elementary Analysis (%) Upper column: Calcd. Lower column: Found |
|---|---|---|---|---|---|---|
| 80 | 2',4'-(Cl)$_2$ | OEt | Pale brown crystals | 82~84 | $C_{10}H_{14}Cl_2O_4$ | C 60.49 H 3.74<br>C 60.57 H 3.66 |
| 81 | 2',4'-(Cl)$_2$ | N (Et)$_2$ | Pale brown crystals | 164~167 | $C_{21}H_{19}Cl_2NO_3$ | C 62.38 H 4.73 N 3.46<br>C 62.55 H 4.67 N 3.45 |

Examples of manufacture of some of the above compounds prepared by other methods than above are given hereunder.

Thus, the compounds of Example 58 (3-methyl-4-pentylflavone-8-carboxylic acid) were manufactured by the following routes (a) or (b), too.

(a) To 19.4 grams of 2-propionyloxybenzoic acid was added 100 ml of carbon disulfide and then 13.3 grams of aluminum chloride (anhydrous) was added thereto and the mixture was stirred until the formation of hydrochloric acid at room temperature ceased. The solvent was then evaporated therefrom, the residue was heated at 150 to 160°C for 4 hours, cooled, the reaction mixture was decomposed with ice and concentrated hydrochloric acid, and extracted with ethyl acetate. The solvent was evaporated from the extract, and the residue was recrystallized from toluene to give 10 grams of 3-propionylsalicyclic acid, colorless needles, melting point 122 to 124°C. To 1.9 grams of the 3-propionylsalicyclic acid were added 5 grams of p-pentylbenzoic acid (sodium salt) and 20 grams of p-pentylbenzoic anhydride and the mixture was heated at 180 to 190°C for 6 hours. Then it was heated to reflux for additional one hour with 15 grams of potassium hydroxide, 50 ml of ethanol and 20 ml of water. The solvent was evaporated therefrom in vacuo, water was added to the residue, the mixture was acidified with 10% hydrochloric acid, and crystals appeared thereby were collected by filtration. The crystals were then recrystallized from a mixture of methanol and chloroform to give 1 gram of the title compound, colorless needles, melting point 202 to 205°C.

(b) Another route to manufacture the same 3-methyl-4-pentylflavone-8-carboxylic acid is given. Thus, to 10 grams of 2-hydroxy-3-propenylpropiophenone were added 20 ml of glacial acetic acid, 10.7 grams of acetic anhydride and 2 drops of hydrochloric acid and the mixture was stirred at 80 to 90°C for 13 hours. The reaction solution was poured into 100 ml of cold water and stirred for 1 hour. The solution was then extracted with benzene, the solvent was evaporated from the extract, and the residue was distilled in vacuo to give 9.7 grams of 2-acetoxy-3-propenylpropiophenone, b.p. 147 to 152°C/8 mmHg. The 2-acetoxy-3-propenylpropiophenone (12 grams) was dissolved in 12 ml of glacial acetic acid, 12 ml of water was added thereto, and then 33 grams of potassium permanganate was gradually added thereto during 2 hours. Temperature of the mixture was maintained at below 15°C throughout. After the reaction, 33 grams of sodium bisulfite was added to decomposed manganese dioxide. Then 60 ml of concentrated hydrochloric acid was added and the mixture was extracted with chloroform. The solvent was evaporated from the extract and the residue was heated to reflux for 1 hour with 20 ml aqueous solution (10%) of sodium hydroxide and 20 ml of methanol. After cooling, 35 ml of concentrated hydrochloric acid was added thereto, crystals separated out therefrom were collected by filtration, and recrystallized from toluene to give 6.6 grams of 3-propionylsalicyclic acid, melting point 122 to 124°C, colorless needles. To a mixture of 2 grams of 3-propionylsalicyclic acid and 6.5 grams of p-pentylbenzoyl chloride was added 7.4 grams of sodium p-pentylbenzoate with stirring and the mixture was heated at 185 to 195°C for 6 hours with stirring. After cooling, the reaction mixture was dissolved in 20 ml of 10% sodium bicarbonate solution and 20 ml of benzene. To an aqueous layer was added 3 ml of concentrated hydrochloric acid with cooling, crystals separated out therefrom were collected by filtration, washed with water, and recrystallized from a mixture of methanol and chloroform to give 1.2 grams of the desired product, colorless needles, melting point 202 to 206°C.

The compound of Example 59 was synthesized by the following way also.

Thus, 2.2 grams of 3-propionylsalicyclic acid ethyl ester was dissolved in 50 ml of dimethyl formamide, the mixture was stirred at room temperature for 1 hour with 0.48 gram of sodium hydride, then 2.1 grams of p-pentylbenzoyl chloride was gradually dropped therein with ice cooling, the mixture was then kept at room temperature, stirred for 1 hour, warmed at 60°C for 3 hours with 0.48 gram of 50% sodium hydride, poured over into ice water, and the mixture was made slightly acidic with 10% hydrochloric acid. The mixture was extracted with ethyl acetate, the solvent was evaporated from the extract, 10 ml of 38% ethanolic hydrochloric acid was added to the resulting oil, the mixture was allowed to stand for 30 minutes at room temperature, then poured into water, extracted with ethyl acetate, the solvent was evaporated

from the extract, and the residue was recrystallized from n-hexane to give 1.4 grams of desired ethyl 3-methyl-4'-pentylflavone-8-carboxylate, colorless needles, melting point 41 to 42°C.

**Claims**

1. 3-Methylflavone derivatives represented by the following general formula (I)

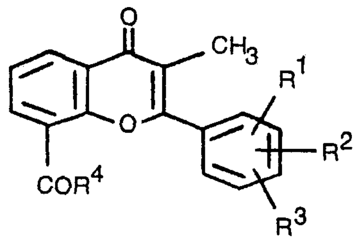

(I)

in which $R^1$, $R^2$ and $R^3$ are same or different and are hydrogen, halogen, alkyl with one to ten carbons, or alkoxy with one to four carbons; $R^4$ is hydroxyl, alkoxy with one to three carbons, hydroxyalkyloxy with one to three carbons or

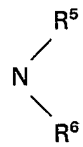

(wherein $R^5$ is hydrogen or alkyl with one to seven carbons; $R^6$ is alkyl with one to seven carbons or cycloalkyl with five to seven members; or $R^5$ and $R^6$ may be joined to form, together with the nitrogen atom to which they are joined, a five- to seven-membered ring, and salts thereof, except the compounds where $R^1$, $R^2$ and $R^3$ are hydrogen and, at the same time, $R^4$ is hydroxyl or ethoxy.

2. Antiinflammatory and/or antiallergic agent consisting, as main ingredient(s) of one or more of the compounds represented by the following general formula (I):

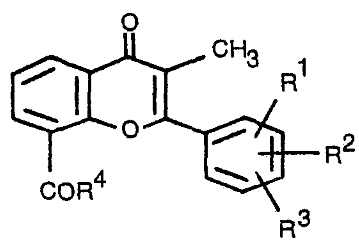

(I)

in which $R^1$, $R^2$ and $R^3$ are same or different and are hydrogen, halogen, alkyl with one to ten carbons, or alkoxy with one to four carbons; $R^4$ is hydroxyl, alkoxy with one to three carbons, hydroxyalkyloxy with one to three carbons or

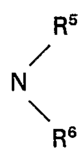

(wherein $R^5$ is hydrogen or alkyl with one to seven carbons; $R^6$ is alkyl with one to seven carbons or cycloalkyl with five to seven members; or $R^5$ and $R^6$ may be joined to form, together with the nitrogen atom to which they are joined, a five-to-seven-membered ring, and salts thereof.

3. A method of manufacturing a compound represented by the following formula (VIII):

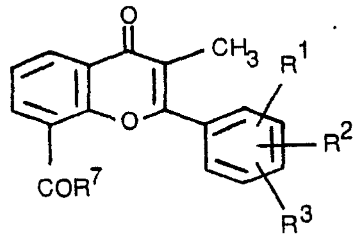

(VIII)

in which $R^1$, $R^2$ and $R^3$ are the same as already defined in claim 1; $R^7$ is hydroxyl, alkoxy with 1 to 3 carbon atom(s), hydroxyalkoxy with 1 to 3 carbon atom(s), or

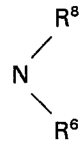

(in which $R^8$ is alkyl with 1 to 7 carbon atom(s); $R^6$ is alkyl with 1 to 7 carbon atom(s) or cycloalkyl with 5 to 7 members; $R^8$ and $R^6$ may form a ring having 5 to 7 members together with a nitrogen atom) by the reaction of a compound of the formula (V):

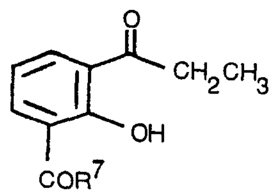

(V)

(in which $R^7$ is the same as already defined) with compounds of the formulae (VI) and (VII):

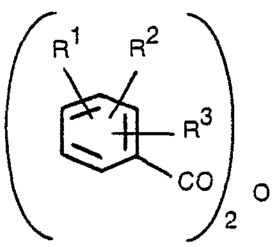

(VI)

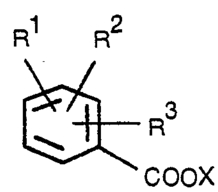

(VII) (X est un metal alcalin)

(in which $R^1$, $R^2$ and $R^3$ are the same as already defined).

4. A method of manufacturing a compound represented by the formula (VIII); of claim 3, characterized by the reaction of a compound of the formula (V) of claim 3 with the compounds of the formulae (IX) and (VII), the latter being identical with that in claim 3,

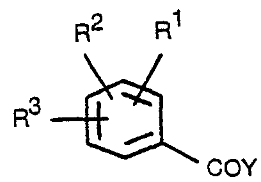

(IX)

in which $R^1$, $R^2$ and $R^3$ are the same as already defined and Y is halogen.

5. A method of manufacturing a compound represented by the formula (X):

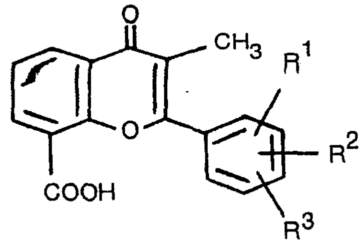

(X)

in which $R^1$, $R^2$ and $R^3$ are the same as already defined, characterized in that 2-hydroxy-3-propenylpropiophenone is made to react with a compound of the formula (IX) of claim 4, in which Y is chlorine and the resulting compound (XI) is subjected to an oxidation to afford the compound (X) in the manner illustrated by the following formulae:

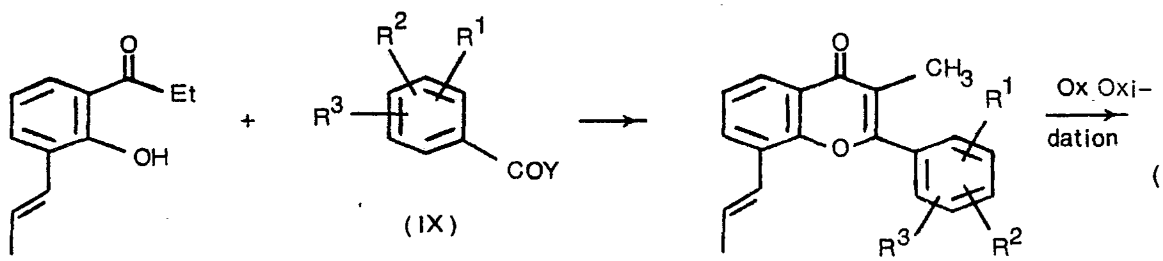


(IX) (XI) (X)

6. A method of manufacturing a compound of the formula (I) of claim 1, characterized in that the compound of the formula (VIII) of claim 3 is esterified or is made to react with a compound of the formula

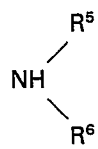

where $R^5$ is hydrogen or alkyl with 1 to 7 carbon atom(s) and $R^6$ is alkyl with 1 to 7 carbon atom(s) or cycloalkyl with 5 to 7 members; $R^5$ and $R^6$ may form a ring of 5 to 7 members together with a nitrogen atom.

7. A method of manufacturing a compound of the formula (VIII) of claim 3, characterized in that a compound of the formula (XII)

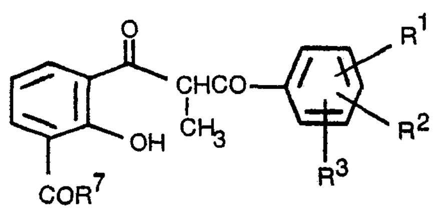

(XII)

in which $R^1$, $R^2$ and $R^3$ are the same as already defined and $R^7$ is also the same as already defined, is subjected to a ring closure reaction.

8. A method of manufacturing a compound of the formula (I) of claim 1, characterized in that the compound of the formula (VIII) of claim 3 is hydrolyzed and, if necessary, further made to react with a compound of a formula

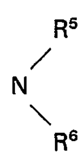

(the meanings of $R^5$ and $R^6$ are the same as those already defined) or the compound of the formula (VIII) of claim 3 is directly made to react with a compound of a formula

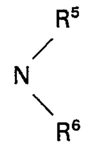

**Patentansprüche**

1. 3-Methylflavon-Derivate der folgenden allgemeinen Formel (I):

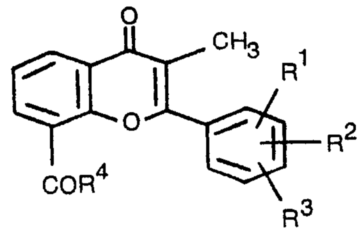

(I)

worin $R^1$, $R^2$ und $R^3$ gleich oder verschieden sind und Wasserstoff, Halogen, Alkyl mit 1 bis 10 Kohlenstoffatomen, oder Alkoxy mit 1 bis 4 Kohlenstoffatomen bedeuten; $R^4$ Hydroxyl, Alkoxy mit 1 bis 3 Kohlenstoffatomen, Hydroxyalkyloxy mit 1 bis 3 Kohlenstoffatomen oder

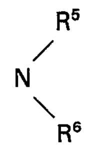

ist, worin $R^5$ Wasserstoff oder Alkyl mit 1 bis 7 Kohlenstoffatomen ist; $R^6$ Alkyl mit 1 bis 7 Kohlenstoffatomen oder Cycloalkyl mit 5 bis 7 Ringgliedern ist; oder $R^5$ und $R^6$ zusammen mit dem Stickstoffatom, an das sie gebunden sind, einen 5- bis 7-gliedrigen Ring bilden, und Salze davon, ausgenommen die Verbindungen, worin $R^1$, $R^2$ und $R^3$ Wasserstoff sind und gleichzeitig $R^4$ Hydroxyl oder Ethoxy ist.

2. Antiinflammatorisches und/oder antiallergisches Mittel, dessen Hauptbestandteil(e) eine oder mehrere der Verbindungen der folgenden allgemeinen Formel (I) ist:

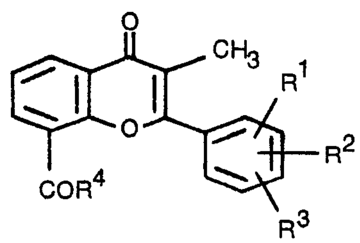

(I)

worin $R^1$, $R^2$ und $R^3$ gleich oder verschieden sind und Wasserstoff, Halogen, Alkyl mit 1 bis 10 Kohlenstoffatomen, oder Alkoxy mit 1 bis 4 Kohlenstoffatomen bedeuten; $R^4$ Hydroxyl, Alkoxy mit 1 bis 3 Kohlenstoffatomen, Hydroxyalkyloxy mit 1 bis 3 Kohlenstoffatomen oder

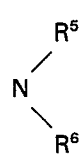

ist, worin $R^5$ Wasserstoff oder Alkyl mit 1 bis 7 Kohlenstoffatomen ist; $R^6$ Alkyl mit 1 bis 7 Kohlenstoffatomen oder Cycloalkyl mit 5 bis 7 Ringgliedern ist; oder $R^5$ und $R^6$ zusammen mit dem Stickstoffatom, an das sie gebunden sind, einen 5- bis 7-gliedrigen Ring bilden, und Salze davon.

3. Verfahren zur Herstellung einer Verbindung der folgenden Formel (VIII)

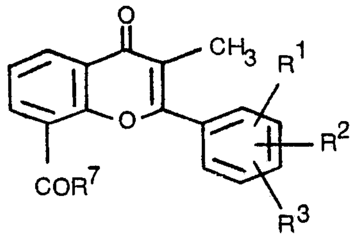

(VIII)

worin $R^1$, $R^2$ und $R^3$ die gleiche Bedeutung wie in Anspruch 1 besitzen; $R^7$ Hydroxyl, Alkoxy mit 1 bis 3 Kohlenstoffatomen, Hydroxyalkoxy mit 1 bis 3 Kohlenstoffatomen, oder

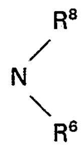

ist, worin $R^8$ Alkyl mit 1 bis 7 Kohlenstoffatomen, $R^6$ Alkyl mit 1 bis 7 Kohlenstoffatomen oder Cycloalkyl mit 5 bis 7 Ringgliedern ist; oder $R^8$ und $R^6$ zusammen mit einem Stickstoffatom einen Ring mit 5 bis 7 Gliedern bilden können, durch Umsetzung einer Verbindung der Formel (V)

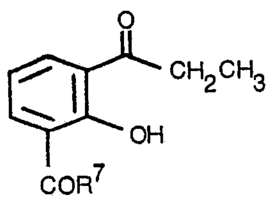

(V)

worin $R^7$ die vorstehend definierte Bedeutung besitzt, mit Verbindungen der Formeln (VI) und (VII)

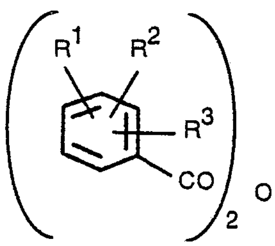

(VI)

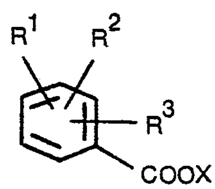

(VII) (X ist un metal alcalin)

worin $R^1$, $R^2$ und $R^3$ die vorstehend definierte Bedeutung besitzen.

4. Verfahren zur Herstellung einer Verbindung der Formel (VIII) gemäß Anspruch 3, dadurch gekennzeichnet, daß man eine Verbindung der Formel (V) gemäß Anspruch 3 mit den Verbindungen der Formeln (IX) und (VII) umsetzt, wobei (VII) identisch mit (VII) gemäß Anspruch 3, und (IX)

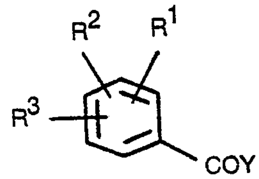

(IX)

ist, worin $R^1$, $R^2$ und $R^3$ die bereits definierte Bedeutung besitzen und Y Halogen ist.

5. Verfahren zur Herstellung einer Verbindung der Formel (X)

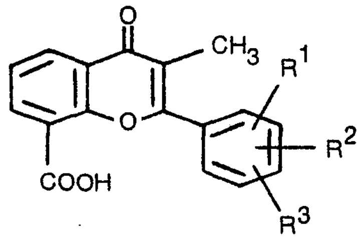

(X)

worin $R^1$, $R^2$ und $R^3$ die bereits definierte Bedeutung besitzen, dadurch gekennzeichnet, daß man 2-Hydroxy-3-propenyl-propiophenon mit einer Verbindung der Formel (IX) gemäß Anspruch 4 zur Reaktion bringt, worin Y Chlor ist, und die resultierende Verbindung (XI) einer Oxidation unterwirft, um die Verbindung (X) in der durch die folgenden Formeln dargestellten Weise zu erhalten:

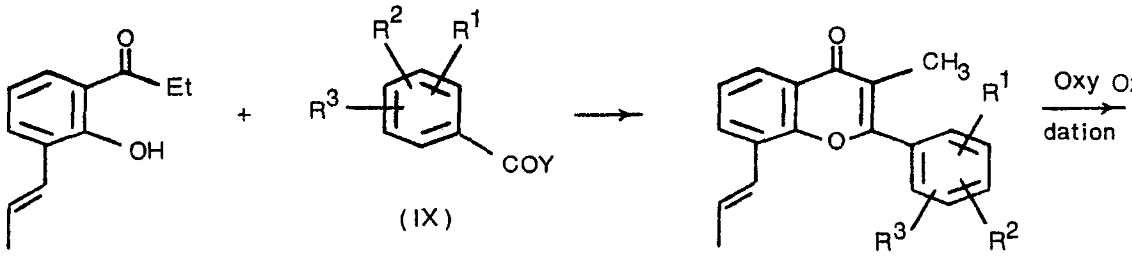


(IX)

(X)

(XI)

6. Verfahren zur Herstellung einer Verbindung der Formel (I) gemäß Anspruch 1, dadurch gekennzeichnet, daß die Verbindung der Formel (VIII) gemäß Anspruch 3 verestert oder mit einer Verbindung der Formel

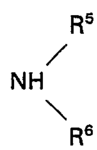

umgesetzt wird, worin $R^5$ Wasserstoff oder Alkyl mit 1 bis 7 Kohlenstoffatomen ist, und $R^6$ Alkyl mit 1 bis 7 Kohlenstoffatomen oder Cycloalkyl mit 5 bis 7 Ringgliedern ist; oder $R^5$ und $R^6$ zusammen mit einem Stickstoffatom einen 5- bis 7-gliedrigen Ring bilden können.

7. Verfahren zur Herstellung einer Verbindung der Formel (VIII) gemäß Anspruch 3, dadurch gekennzeichnet, daß man eine Verbindung der Formel (XII)

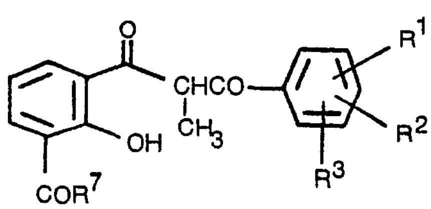

(XII)

worin $R^1$, $R^2$ und $R^3$ die bereits definierte Bedeutung besitzen und $R^7$ auch die gleiche bereits definierte Bedeutung besitzt, einer Ringschlußreaktion unterwirft.

8. Verfahren zur Herstellung einer Verbindung der Formel (I) gemäß Anspruch 1, dadurch gekennzeichnet, daß die Verbindung der Formel (VIII) gemäß Anspruch 3 hydrolysiert wird und, wenn erforderlich, weiters mit einer Verbindung der Formel

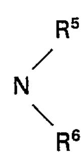

umgesetzt wird, worin die Bedeutungen von $R^5$ und $R^6$ die gleichen sind wie bereits definiert, oder die Verbindung der Formel (VIII) gemäß Anspruch 3 direkt mit einer Verbindung der Formel

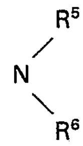

umgesetzt wird.

**Revendications**

1. Dérivés de la 3-méthylflavone répondant à la formule générale (I) suivante:

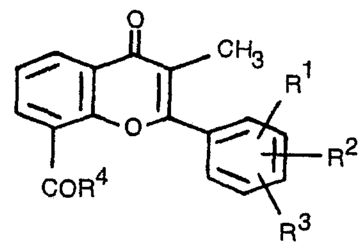

(I)

dans laquelle $R^1$, $R^2$ et $R^3$ sont identiques ou différents et sont l'hydrogène, un halogène, un groupe alkyle en $C_1$ à $C_{10}$ ou un groupe alcoxy en $C_1$ à $C_4$; $R^4$ est un groupe hydroxyle, alcoxy en $C_1$ à $C_3$, hydroxyalkyloxy en $C_1$ à $C_3$ ou

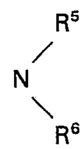

(où $R^5$ est l'hydrogène ou un groupe alkyle en $C_1$ à $C_4$; $R^6$ est un groupe alkyle en $C_1$ à $C_7$ ou un groupe cycloalkyle ayant 5 à 7 éléments; ou bien $R^5$ et $R^6$ peuvent se réunir pour former avec l'atome d'azote auquel ils sont liés, un cycle à 5 à 7 maillons et, leurs sels, excepté les composés dans lesquels $R^1$, $R^2$ et $R^3$ sont l'hydrogène et, en même temps, $R^4$ est un groupe hydroxyle ou éthoxy.

2. Agent anti-inflammatoire et/ou anti-allergique contenant, comme ingrédients principaux un ou plusieurs des composés répondant à la formule générale (I) suivante:

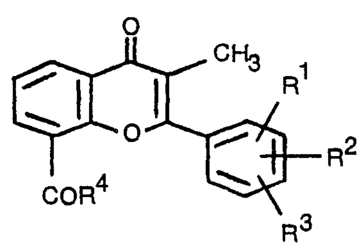

(I)

dans laquelle $R^1$, $R^2$ et $R^3$ sont identiques ou différents et sont l'hydrogène, un halogène, un groupe alkyle en $C_1$ à $C_{10}$ ou un groupe alcoxy en $C_1$ à $C_4$; $R^4$ est un groupe hydroxyle, alcoxy en $C_1$ à $C_3$, hydroxyalkyloxy en $C_1$ à $C_3$ ou

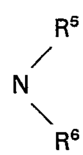

(où $R^5$ est l'hydrogène ou un groupe alkyle en $C_1$ à $C_7$, $R^6$ est un groupe alkyle en $C_1$ à $C_7$ ou un groupe thioalkyle ayant 5 à 7 maillons, ou bien $R^5$ et $R^6$ peuvent s'unir pour former avec l'atome d'azote auquel ils sont liés, un cycle à 5 à 7 maillons et leurs sels.

3. Procédé de préparation d'un composé répondant à la formule générale (VII):

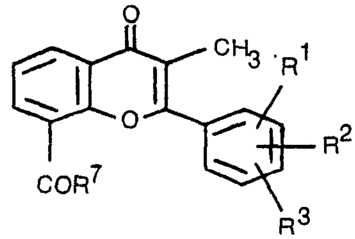

(VIII)

dans laquelle $R^1$, $R^2$ et $R^3$ sont tels que déjà définis dans la revendication 1; $R^7$ est un groupe hydroxyle, alcoxy en $C_1$ à $C_3$, hydroxyalcoxy en $C_1$ à $C_3$ ou

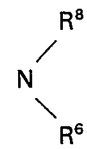

(où $R^8$ est un groupe alkyle en $C_1$ à $C_7$); $R^6$ est un groupe alkyle en $C_1$ à $C_7$ ou cycloalkyle en $C_5$ à $C_7$; $R^8$ et $R^6$ peuvent former un cycle ayant 5 à 7 maillons en même temps qu'un atome d'azote) par la réaction d'un composé répondant à la formule (V):

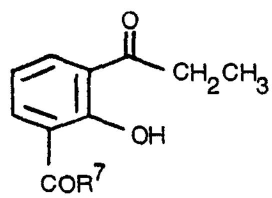

(V)

(dans laquelle $R^7$ est tel que défini précédemment) avec des composés répondant aux formules (VI) et (VII).

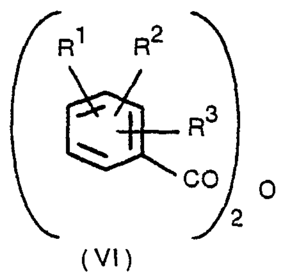

(VI)

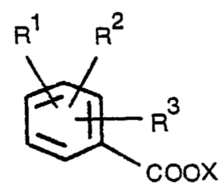

(VII) (X est un métal alcalin)

(dans laquelle $R^1$, $R^2$ et $R^3$ sont tels que définis précédemment).

4. Procédé de préparation d'un composé représenté par la formule (VIII) de la revendication 3, caractérisé par la réaction d'un composé répondant à la formule (V) de la revendication 3 avec les composés répondant aux formules (IX) et (VII), ce dernier étant identique à celui de la revendication 3,

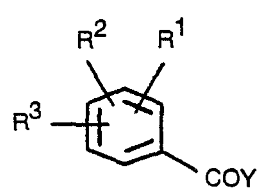

(IX)

dans laquelle $R^1$, $R^2$ et $R^3$ sont tels que définis précédemment et Y est un halogène.

5. Procédé de préparation d'un composé représenté par la formule (X):

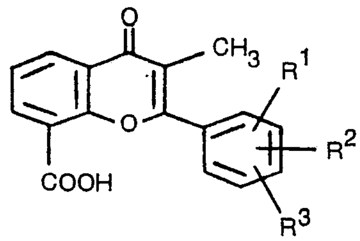

(X)

dans laquelle $R^1$, $R^2$ et $R^3$ sont tels que définis précédemment, caractérisé en ce qu'on fait réagir la 2-hydroxy-3-propénylpropiophénone avec un composé répondant à la formule (IX) de la revendication 4, dans laquelle Y est le chlore et le composé (XI) obtenu est soumis à une oxydation pour donner le composé (X) de la manière représentée dans les formules suivantes:

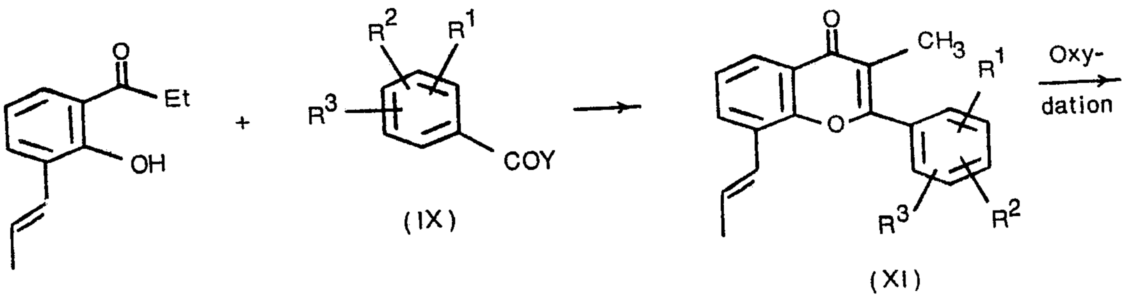


(IX) (XI) (X)

6. Procédé de préparation d'un composé répondant à la formule (I) de la revendication 1, caractérisé en ce que le composé répondant à la formule (VIII) de la revendication 3 est estérifié ou mis à réagir avec un composé répondant à la formule

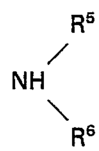

dans laquelle $R^5$ est l'hydrogène, ou un groupe alkyle en $C_1$ à $C_7$, et $R^6$ est un groupe alkyle en $C_1$ à $C_7$ ou cycloalkyle à 5 à 7 maillons, $R^5$ et $R^6$ peuvent former un cycle à 5 à 7 maillons avec un atome d'azote.

7. Procédé de préparation d'un composé répondant à la formule (VIII) de la revendication 3, caractérisé en ce qu'on soumet à une réaction de fermeture de cycle un composé répondant à la formule (XII):

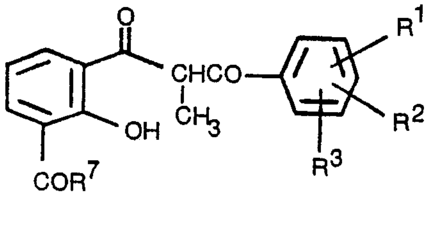

(XII)

dans laquelle $R^1$, $R^2$ et $R^3$ sont tels que définis précédemment et $R^7$ est aussi tel que défini précédemment.

8. Procédé de préparation d'un composé répondant à la formule (I) de la revendication 1, caractérisé en ce qu'on hydrolyse le composé répondant à la formule (VIII) de la revendication 3, ou, si nécessaire, on le fait encore réagir avec un composé répondant à la formule

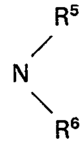

(les significations de $R^5$ et $R^6$ sont les mêmes que celles déjà définies) ou on fait réagir directement le composé répondant à la formule (VIII) de la revendication 3 avec un composé répondant à la formule

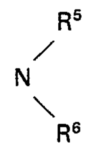 .